# EUROPEAN PATENT APPLICATION

(11) **EP 4 792 823 A1**
(43) Date of publication of application: **19.08.2026**
(21) Application number: 25158140.1
(22) Date of filing: 14.02.2025
(51) Int. Cl.: A61K 38/16, A61P 31/14

(54) **ANTIVIRAL POLYPEPTIDES AGAINST CORONAVIRUS**

(71) Applicant: Universität Leipzig, 04109 Leipzig (DE)
(72) Inventor: HOFFMANN, Ralf, 04109 Leipzig (DE); SCHWARZE, Mandy, 04109 Leipzig (DE); KRIZSAN, Andor, 04109 Leipzig (DE)
(74) Representative: Vossius & Partner Patentanwälte Rechtsanwälte mbB

(57) **Abstract**

The present invention is in the field of virology. The present invention provides polypeptides that blocks complex formation between a surface protein of a coronavirus and a receptor of a target cell, wherein the polypeptide is derived from the N-terminal region of the spike (S) protein receptor binding domain (RBD) of a coronavirus, preferably wherein the polypeptide binds to the receptor of the target cell. In particular, the present invention relates to a polypeptide for use in treatment and/or prevention of coronavirus infection, comprising an amino acid sequence according to SEQ ID NO: 4 or an amino acid sequence of at least 60% sequence identity to SEQ ID NO: 4.

## Description

The present invention is in the field of virology. The present invention provides polypeptides that blocks complex formation between a surface protein of a coronavirus and a receptor of a target cell, wherein the polypeptide is derived from the N-terminal region of the spike (S) protein receptor binding domain (RBD) of a coronavirus, preferably wherein the polypeptide binds to the receptor of the target cell. In particular, the present invention relates to a polypeptide for use in treatment and/or prevention of coronavirus infection, comprising an amino acid sequence according to SEQ ID NO: 4 or an amino acid sequence of at least 60% sequence identity to SEQ ID NO: 4.

Severe acute respiratory syndrome coronavirus 2 (SARS-CoV-2) emerged in China in 2019 causing coronavirus disease 2019 (COVID-19). The ancestral SARS-CoV-2 strain (hereafter also referred to as wild-type, WT, or original Wuhan strain) infects the human respiratory system, causing mostly mild flu-like symptoms, such as cough and fever, but a few percent of patients experience more severe cases with pneumonia and respiratory distress, and even death (Huang et al. (2020). The Lancet, 10223, S. 497-506; Liu et al. (2020). Chinese Medical Journal, 9, S. 1025-1031; Wang et al. (2020). JAMA, 11, S. 1061). As of January 7, 2024, the World Health Organization (WHO) has counted more than 777 million reported COVID-19 cases and more than 7 million reported COVID-19 deaths (WHO COVID-19 dashboard). Furthermore, long-lasting effects such as loss of taste, pain, and increased respiratory distress (long COVID) have been documented (O'Laughlin et al. (2022). PloS one, 3, e0264260; Han et al. (2022). Influenza and other respiratory viruses, 4, S. 680-689; Gottlieb et al. (2023). Open forum infectious diseases, 7, ofad277). During the pandemic, several variants of concern (VOCs), such as the alpha, beta, gamma, delta (kappa), and various omicron variants, have evolved from the original Wuhan strain, which partially escape the immune response induced by previous SARS-CoV-2 infections or vaccination efforts starting in 2020, such as mRNA vaccines from Moderna (mRNA-1273) and BioNTech (BNT162b2) (van Vo et al. (2022). Microorganisms, 3, S. 598). For example, a new wave of infections and reinfections caused by the omicron variant BA.1 was reported in late 2021 (Kim et al. (2022). bioRxiv). This was partly due to the fact that the vaccination did not stimulate sufficient levels of neutralizing antibodies (only 60-80%) required for full immunity, even after the third (booster) vaccination (Schwarze et al. (2022). Frontiers in immunology, 13, S. 915034). To date, other omicron variants with different mutations have evolved, such as BA.4/5, which peaked in summer 2022 and continued to spread in the summer of 2023, while other variants such as XBB, JN.1 and KP.3 have been on the rise (Akash et al. (2023). International Journal of Surgery, 4, S. 658-659; Tallei et al. (2023). Reviews in medical virology, 1, e2391; van Vo et al. (2022). Microorganisms, 3, S. 598). For these reasons, and in anticipation of future zoonotic coronavirus infections, it remains important to develop further therapeutic approaches, especially for individuals with compromised or suppressed immune systems.

As laid out above, the main challenges of the current options for treatment and/or prevention of coronavirus infection is the continuing evolvement of novel virus variants and their escape from the patient's immune response induced by previous coronavirus infections or vaccination efforts.

Accordingly, the technical problem underlying the present invention is the provision of improved means and methods for antiviral therapeutic approaches against coronavirus infections, in particular against SARS-CoV-2 infections.

The above technical problem is solved by the provisions of the embodiments as characterized in the claims and as described herein below.

The invention is, *inter alia,* characterized by the following items:
The present invention relates generally to a polypeptide that blocks complex formation between a surface protein of a coronavirus and a receptor of a target cell, wherein the polypeptide is derived from the N-terminal region of the RBD of the spike (S) protein of a coronavirus, preferably wherein the polypeptide binds to the receptor of the target cell.
1. A polypeptide for use in treatment and/or prevention of coronavirus infection, comprising an amino acid sequence according to SEQ ID NO: 4 or
   an amino acid sequence of at least 80% sequence identity to SEQ ID NO: 4.
2. The polypeptide for use according to item 1, comprising amino acid substitutions relative to SEQ ID NO: 4 selected from:
   (i) K26N,
   (ii) K26T,
   (iii) D14N, R17S, K26N, or
   (iv) D14N, R17S, K26T.
3. The polypeptide for use according to any one of items 1-2, wherein the amino acid sequence is selected from the group of SEQ ID NOs: 7, 6, 5, and 8, preferably SEQ ID NO: 7.
4. The polypeptide for use according to any one of items 1-3, wherein the polypeptide is C-terminally modified, such as amidated, and/or N-terminally modified, such as acetylated.
5. The polypeptide for use according to any one of items 1-4, wherein the polypeptide blocks complex formation between a surface protein of the coronavirus and a receptor of a target cell.
6. The polypeptide for use according to any one of items 1-5, wherein the polypeptide binds to a receptor of a target cell.
7. The polypeptide for use according to any one of items 5-6, wherein the receptor of a target cell is membrane receptor of a target cell.
8. The polypeptide for use according to item 7, wherein the membrane receptor of a target cell is ACE-2 receptor.
9. The polypeptide for use according to any one of items 1-8, wherein the polypeptide prevents cell penetration of the coronavirus.
10. The polypeptide for use according to any one of items 1-9, wherein the coronavirus is selected from the group of SARS-CoV-2, SARS-CoV-1, HCoV-NL63, and related Coronaviridae, preferably SARS-CoV-2.
11. The polypeptide for use according to item 10, wherein the SARS-CoV-2 is a SARS-CoV-2 variant selected from the group of ancestral strain, alpha, beta, gamma, delta (kappa), omicron and subvariants thereof.
12. The polypeptide for use according to item 11, wherein the subvariant of the SARS-CoV-2 variant omicron is selected from the BA.1, BA.2, BA.3, BA.3, BA.4, XBB, JN.1, KP.3, BQ, XE and KP.2.
13. The polypeptide for use according to any one of items 1-12, wherein the polypeptide is to be administered in a therapeutically effective amount.
14. The polypeptide for use according to item 13, wherein the therapeutically effective amount is in the range of about 33 to about 800 mg/L, equivalent to about 10 to about 244 µM.
15. The polypeptide for use according to any one of items 1-14, wherein the polypeptide is to be administered parenterally or orally.
16. The polypeptide for use according to items 15, wherein the parenteral administration is by a spray, such as a nasal or oral spray, by inhalation, such as vapor, powder, or aerosol inhalation, or by injection, preferably by nasal spray or aerosol inhalation.
17. The polypeptide for use according to any one of items 1-16, wherein the polypeptide is to be administered in combination with one or more further antiviral agent(s).
18. The polypeptide for use according to item 17, wherein the one or more further antiviral agent(s) is/are selected from the group of: mRNA vaccines, adenovirus vector vaccines, inactivated virus vaccines, subunit vaccines, monoclonal antibody therapies and other small molecule treatments, preferably, wherein the one or more further antiviral agent(s) is selected from the group of Remdesivir, Nirmatrelvir, Ritonavir, Dexamethasone, and Tocilizumab.
19. The polypeptide for use according to item 18, wherein the further antiviral agent is an antibody.
20. The polypeptide for use according to item 19, wherein the antibody binds a coronavirus.
21. The polypeptide for use according to items 20, wherein the antibody binds the spike (S) protein of the coronavirus.
22. The polypeptide for use according to item 21, wherein the antibody binds the RBD of the coronavirus spike (S) protein.
23. The polypeptide for use according to any one of items 20-22, wherein the coronavirus is selected from the group of SARS-CoV-2, SARS-CoV-1, HCoV-NL63, and related Coronaviridae, preferably SARS-CoV-2.
24. The polypeptide for use according to item 19, wherein the antibody binds the ACE-2 receptor.
25. The polypeptide for use according to any one of items 17-24, wherein the polypeptide and the one or more further antiviral agent(s) are to be administered simultaneously or sequentially during the course of the same treatment period.
26. The polypeptide for use according to any one of items 17-25, wherein the polypeptide and the one or more further antiviral agent(s) are administered once or multiple times during the same treatment period.
27. A polypeptide, comprising
   an amino acid sequence selected from the group of SEQ ID NOs: 7, 6, 5, 8,
   preferably SEQ ID NO: 7, or
   an amino acid sequence of at least 80% sequence identity to SEQ ID NOs: 7, 6, 5, 8.
28. The polypeptide according to item 27, comprising amino acid substitutions relative to SEQ ID NO: 4 selected from:
   (i) K26N,
   (ii) K26T,
   (iii) D14N, R17S, K26N, or
   (iv) D14N, R17S, K26T.
29. The polypeptide according to any one of items 27-28, wherein the polypeptide is C-terminally modified, such as amidated, and/or N-terminally modified, such as acetylated.
30. The polypeptide according to any one of items 27-29, wherein the polypeptide blocks complex formation between a surface protein of a coronavirus and a receptor of a target cell.
31. The polypeptide according to any one of items 27-30, wherein the polypeptide binds to a receptor of a target cell.
32. The polypeptide according to any one of items 30-31, wherein the receptor of a target cell is membrane receptor of a target cell.
33. The polypeptide according to item 32, wherein the membrane receptor of a target cell is ACE-2 receptor.
34. The polypeptide according to any one of items 27-33, wherein the polypeptide prevents cell penetration of the coronavirus.
35. The polypeptide according to item 34, wherein the coronavirus is selected from the group of SARS-CoV-2, SARS-CoV-1, and HCoV-NL63, preferably SARS-CoV-2.
36. The polypeptide according to item 35, wherein the SARS-CoV-2 is a SARS-CoV-2 variant selected from the group of ancestral strain, alpha, beta, gamma, delta (kappa), omicron and subvariants thereof.
37. The polypeptide according to item 36, wherein the subvariant of the SARS-CoV-2 variant omicron is selected from the BA.1, BA.2, BA.3, BA.3, BA.4, XBB, JN.1, KP.3, BQ, XE and KP.2.
38. The polypeptide according to any one of items 27-37, wherein the polypeptide is to be administered in a therapeutically effective amount.
39. The polypeptide according to item 38, wherein the therapeutically effective amount as an antiviral is in the range of 33 to 800 mg/L, equivalent to 10 to 244 µM.
40. The polypeptide according to any one of items 27-39, wherein the polypeptide is to be administered parenterally or orally.
41. The polypeptide according to items 40, wherein the parental administration is by a spray, such as a nasal or oral spray, by inhalation, such as vapor, powder, or aerosol inhalation, or by injection, preferably by nasal spray or aerosol inhalation.
42. The polypeptide according to any one of items 27-41, wherein the polypeptide is to be administered in combination with one or more further antiviral agent(s).
43. The polypeptide according to item 42, wherein the one or more further antiviral agent(s) is/are selected from the group of: mRNA vaccines, monoclonal antibody therapies or other small molecule treatments, preferably, wherein the one or more further antiviral agent(s) is selected from the group of Remdesivir, Nirmatrelvir, Ritonavir, Dexamethasone, and Tocilizumab.
44. The polypeptide according to item 42, wherein the further antiviral agent is an antibody.
45. The polypeptide according to item 44, wherein the antibody binds a coronavirus.
46. The polypeptide according to items 45, wherein the antibody binds the spike (S) protein of the coronavirus.
47. The polypeptide according to items 46, wherein the antibody binds the RBD of the coronavirus spike (S) protein.
48. The polypeptide according to any one of items 45-47, wherein the coronavirus is selected from the group of SARS-CoV-2, SARS-CoV-1, and HCoV-NL63, preferably SARS-CoV-2.
49. The polypeptide according to item 44, wherein the antibody binds the ACE-2 receptor.
50. The polypeptide according to any one of items 42-49, wherein the polypeptide and the one or more further antiviral agent(s) are to be administered simultaneously or sequentially during the course of the same treatment period.
51. The polypeptide according to any one of items 42-50, wherein the polypeptide and the one or more further antiviral agent(s) are administered once or multiple times during the same treatment period.
52. A polypeptide as defined in any one of items 1-26 for use as a medicament.
53. A composition comprising a polypeptide as defined in any one of items 1-26 as an active ingredient.
54. A composition for use as a medicament, comprising a polypeptide as defined in any one of items 1-26 as an active ingredient.
55. A composition for use in treatment and/or prevention of coronavirus infection, comprising a polypeptide as defined in any one of items 1-26 as an active ingredient.
56. A pharmaceutical composition comprising the polypeptide as defined in any one of items 1-26 and a pharmaceutical acceptable carrier, excipient, or diluent.
57. A pharmaceutical composition for use as a medicament, comprising the polypeptide as defined in any one of items 1-26 and a pharmaceutical acceptable carrier, excipient, or diluent.
58. A pharmaceutical combination for use in treatment and/or prevention of coronavirus infection, comprising the polypeptide as defined in any one of items 1-26 and at least one pharmaceutical acceptable carrier, excipient, and/or diluent.
59. Use of the polypeptide as defined in any one of items 1-26 for potentiating the antiviral effect of one or more agent(s), wherein the one or more agent(s) is/are selected from the group of: mRNA vaccines, monoclonal antibody therapies or other small molecule treatments, preferably wherein the one or more agent(s) is/are selected from the group of Remdesivir, Nirmatrelvir, Ritonavir, Dexamethasone, and Tocilizumab.
60. A nucleic acid molecule comprising a gene encoding the polypeptide as defined in any one of items 1-26.
61. A vector encoding the nucleic acid molecule according to item 60.
62. A host cell comprising the nucleic acid molecule according to item 60, or the vector according to item 61.
63. A method for producing a polypeptide comprising the step of a) culturing the host cell according to item 62, under conditions in which the polypeptide is expressed.
64. The method according to item 63, wherein the method further comprises the step of b) isolating the polypeptide from the cultured host cell.
65. A method of inhibiting cell entry of a coronavirus into a target cell comprising the step of contacting the target cell with the polypeptide as defined in any one of item 1-26.
66. The method according to item 65, wherein the target cell is *in vivo, in vitro or ex vivo.*
67. A kit comprising the polypeptide as defined in any one of item 1-26, the composition according to items 53 to 55, the pharmaceutical composition according to items 56 to 58, the nucleic acid molecule according to item 60, the vector according to item 61, or the host cell according to item 62.

The present invention provides a polypeptide that blocks complex formation between a surface protein of a coronavirus and a receptor of a target cell, wherein the polypeptide is derived from the N-terminal region of the RBD of the spike (S) protein of a coronavirus, preferably wherein the polypeptide binds to the receptor of the target cell.

In a first aspect, the present invention relates to a polypeptide for use in treatment and/or prevention of coronavirus infection, comprising an amino acid sequence according to SEQ ID NO: 4 or an amino acid sequence of at least 60% sequence identity to SEQ ID NO: 4.

In a second aspect, the present invention further relates to a polypeptide, comprising an amino acid sequence selected from the group of SEQ ID NOs: 7, 6, 5, 8, or an amino acid sequence of at least 60% sequence identity to SEQ ID NOs: 7, 6, 5, 8.

In a third aspect, the present invention relates to a polypeptide for use as a medicament, comprising an amino acid sequence according to SEQ ID NO: 4 or an amino acid sequence of at least 60% sequence identity to SEQ ID NO: 4.

In further aspects, the present invention relates to a composition, for example, a pharmaceutical composition, comprising a polypeptide of the present invention. The present invention also provides said composition, for example, said pharmaceutical composition, comprising a polypeptide of the present invention as an active ingredient for use as a medicament, or for use in treatment and/or prevention of coronavirus infection.

In another aspect, the present invention teaches the use of a polypeptide of the present invention for potentiating the antiviral effect of one or more agent(s).

In additional aspects, the present invention teaches a nucleic acid molecule comprising a gene encoding a polypeptide of the present invention, a vector comprising said nucleic acid molecule, an organelle comprising said nucleic acid molecule, and a host cell comprising said nucleic acid molecule, the vector comprising said nucleic acid molecule, or an organelle comprising said nucleic acid molecule. The present invention also provides a method for producing a polypeptide of the present invention, as well as a method of inhibiting cell entry of a coronavirus into a target cell.

In yet another aspect, the present invention also relates to a kit comprising a polypeptide of the present invention, a composition comprising a polypeptide of the present invention, a pharmaceutical composition comprising a polypeptide of the present invention, a vector comprising a nucleic acid molecule comprising a gene encoding a polypeptide of the present invention, or a host cell comprising a nucleic acid molecule comprising a gene encoding a polypeptide of the present invention, a vector comprising a nucleic acid molecule comprising a gene encoding a polypeptide of the present invention, or an organelle comprising a nucleic acid molecule comprising a gene encoding a polypeptide of the present invention.

As used herein, "a polypeptide of the present invention", also referred to as "the polypeptides of the present invention" or similar grammatical variations thereof, may relate to the polypeptide as defined in any of the herein mentioned aspects of the present invention, unless specified to the contrary.

In the following the invention is described in more detail.

The present inventors have surprisingly identified a family of polypeptides that can efficiently bind to a target cell receptor, blocking complex formation between a surface protein of a coronavirus and a receptor of a target cell, and thereby preventing target cell penetration of the coronavirus. Accordingly, the present invention provides a polypeptide that blocks complex formation between a surface protein of a coronavirus and a receptor of a target cell, wherein the polypeptide is derived from the N-terminal region of the RBD of the spike (S) protein of a coronavirus, preferably wherein the polypeptide binds to the receptor of the target cell. In context of the present invention, the N-terminal region relates to an amino acid sequence spanning about 100 to about 115 amino acid residues from the N-terminus of a given protein, region, domain, or motif. Accordingly, in context of the present invention, the N-terminal region of the RBD of the spike (S) protein relates to a region between amino acid residue positions, spanning from about 319 to about 429, relative to the full-length S protein (SEQ ID NO: 1).

In context of the present invention, the coronavirus is selected from the group of SARS-CoV-2, SARS-CoV-1, HCoV-NL63, and related Coronaviridae, preferably SARS-CoV-2. In context of the present invention, "related Coronaviridae" refers to any virus selected from the genus Alpha-, Beta-, Gamma-, or Deltacoronavirus. In context of the present invention, variants of a coronavirus are based on and originated from mutations of the ancestral strain of the virus. Accordingly, in context of the present invention, the SARS-CoV-2 is a SARS-CoV-2 variant selected from, but is not limited to, the group of ancestral strain, alpha variant, beta variant, gamma variant, delta (kappa) variant, omicron variant and subvariants thereof. In context of the present invention, the "ancestral strain" herein also referred to as "wild-type", "WT", or "original Wuhan strain", is also know in the art as "Wuhan-Hu-1 strain", "HCoV-19", or "2019-nCoV". The corresponding NCBI and UniProt Taxon ID number is 2697049 (see, e.g., NCBI:txid2697049 or https://www.uniprot.org/taxonomy/2697049). Furthermore, in context of the present invention, subvariants of a coronavirus variant are based on and originated from the mutations of a particular variant, in particular on a variant of concern (VOC). Accordingly, non-limiting examples of a subvariant of the SARS-CoV-2 variant omicron may be selected from, but are not limited to, the subvariants BA.1, BA.2, BA.3, BA.3, BA.4, XBB, JN.1, KP.3, BQ, XE and KP.2. In context of the present invention, the term "variant of concern" or "VOC", particularly in relation to SARS-CoV-2, is a category used for variants of the virus where mutations in their S protein RBD substantially increase binding affinity in RBD-ACE2 complex, while also being linked to rapid spread in populations.

Several classes of polypeptides, such as antimicrobial polypeptides, have already been evaluated for their potential to suppress coronavirus infections, in particular SARS-CoV-2 infection, but with moderate results so far (Wang et al. (2021). ACS infectious diseases, 6, S. 1545-1554; Saini et al. (2022). Microbiological research, 265, S. 127206; Schütz et al., (2020). Advanced Drug Delivery Reviews, 167, 47-65). The receptor binding motif (RBM), encompassing residues N437 to Y508 of the spike (S) protein, represents the interaction site between the receptor binding domain (RBD, residues R319 to F541 of the S protein) and the angiotensin converting enzyme 2 (ACE-2) receptor of human cells. As this binding resembles the first step of the cell infection cycle, the RBM represents the most relevant sequence to consider for inhibition of the RBD-ACE-2 interaction, assuming at least properly induced folding upon binding to ACE-2. RBM-based sequences investigated so far were selected on the basis of reported RBD-ACE-2 structures. The key interacting residues of the RBD in general are the strong binding residues K417, F456, F486, Q493, Q498, and N501 and the weak binding residues Y449, L455, N487, Y489, Y495, T500, and Y505 (Jawad et al. (2021). Journal of chemical information and modeling, 9, S. 4425-4441 Starr et al. (2020). Cell, 182, 1295-1310; Rajpoot et al. (2021). Drugs in R&D, 3, S. 273-283; Biskupek and Gieldon (2024). Int. J. Mol. Sci., 25,679; Liu et al. (2023). Heliyon, 9, e12890; Watson et al. (2020). bioRxiv*,* WO 2021/178971). However, the polypeptides tested so far show only weak inhibitory effects *in vitro* at a high concentration of 100 µmol/L, suggesting a limited therapeutic potential (Rajpoot et al. (2021). Drugs in R&D, 3, S. 273-283). Other studies taught reduced adherence and/or low specificity and selectivity (WO 2023/275060; (Schwarze et al. (2022). Pathogens, 12, S. 1515; Tang et al. (2023). Computers in Biology and Medicine, 173, 18325) as well as lack of inhibiting effect (Loi et al. (2023). Heliyon, 9, e22614) for a variety of polypeptides derived from the RBD. Alternatively, polypeptides have been derived from the ACE-2 receptor protein, more specifically from the binding counterpart region of the RBM, or from the RBM itself, whether native or engineered in modeling studies (Rathod et al. (2020). In silico pharmacology, 1, S. 3; Chitsike et al. (2021). Advances in pharmacological and pharmaceutical sciences, S. 1828792; Mahindra et al. (2021). PloS one, 11, e0260283). Wang *et al.* tested polypeptides of the ACE-2 binding site *in vitro,* of which two polypeptides inhibited S-trimer binding to 293T-ACE2hR cells only at concentrations of ~50 mmol/L, which is far too high for therapeutic applications (Wang et al. (2022). Frontiers in microbiology, 13, S. 910343; Sadremomtaz et al. (2022). Journal of medicinal chemistry, 4, S. 2836-2847; Khater und Nassar (2022). BMC pharmacology & toxicology, 1, S. 91). Sequence 481 to 510 within the RBM has been identified in other studies as the major contributor to ACE-2 recognition, as this region contains 67% of the strong and 71% of the weak binding sites (WO 2023/275060; Mahindra et al. (2021). PloS one, 11, e0260283; Sadremomtaz et al. (2022). Journal of medicinal chemistry, 4, S. 2836-2847). Sandremomtaz and colleagues used microscale thermophoresis (MST) to demonstrate binding between the polypeptides and the cognate receptor, but the polypeptides were physiologically inactive and failed in viral inhibition assays.

The present inventors, however, have surprisingly found a family of polypeptides, derived from the N-terminal region of the RBD of the SARS-CoV-2 S protein, that can efficiently bind to the ACE-2 receptor, blocking the complex formation between the RBD and the human ACE-2 receptor, and thereby preventing target cell penetration of a coronavirus, in particular of SARS-CoV-2.

Accordingly, the present invention provides that a polypeptide of the present invention blocks complex formation between a surface protein of a coronavirus and a receptor of a target cell, wherein the polypeptide is derived from the N-terminal region of the RBD of the spike (S) protein of a coronavirus, as defined herein, preferably wherein the polypeptide binds to the receptor of the target cell. Preferably, a polypeptide of the present invention may exhibit an IC₅₀ concentration of at least 85 µmol/L using the respective coronavirus variant RBD as a competitor for target cell receptor binding. Alternatively, a polypeptide of the present invention may preferably exhibit an inhibition rate (%) of between about 30% and 100%, as determined by the pseudovirus assay described herein below. Means and method for determining the inhibitory effect of a polypeptide of the present invention are known in the art and illustratively, but not limiting, described herein below. The person skilled in the art is aware that the effective inhibition of a polypeptide may optionally be determined by other means and methods, some of which are also further described herein, such as the RBD-ACE2 assay (see, e.g., **Example 1-E).** It is envisaged that the effectiveness of the polypeptide is preferably determined by the determination of the IC₅₀ concentration, preferably by the methods described herein. In a particularly preferred embodiment, the polypeptides of the present invention have the same or essentially the same complex blocking effects/inhibitory effect (e.g., as reflected by IC₅₀ or inhibition rate as described herein) as the polypeptides as defined by any one of SEQ ID NOs: 4, 5, 6, 7 or 8. Accordingly, any polypeptide of the present invention preferably possesses the same or essentially the same inhibitory properties as those explicitly exemplified. "Essentially the same" means that the polypeptides have complex blocking effects/inhibitory effect of about ±10 %, preferably ± 5 %, more preferably ± 1 %, compared to the complex blocking effects/inhibitory effect polypeptides as defined by any one of SEQ ID NOs: 4, 5, 6, 7 or 8.

The present invention is particularly advantageous over existing prevention and/or treatment approaches against SARS-CoV-2 for individuals with compromised or suppressed immune systems, as the present invention does not rely on the immune system to prevent and/or treat a coronavirus infection. Thus, the polypeptides to be used in the present invention are in particular for use in treatment and/or prevention of coronavirus infection in individuals with compromised or suppressed immune systems. The present invention concerns a prevention and/or treatment approach that suppress the initial cycle of coronavirus infection by directly interacting with the target cell receptor, in particular the ACE-2 receptor, and inhibits complex formation with the S protein. The polypeptides of the present invention thus directly prevent cell entry of the coronavirus, such as SARS-CoV-2, SARS-CoV-1, HCoV-NL63, and related Coronaviridae, by preventing its binding to the ACE-2 receptor.

In a first aspect, the present invention provides a polypeptide for use in treatment and/or prevention of coronavirus infection, comprising an amino acid sequence according to SEQ ID NO: 4 or an amino acid sequence of at least 60% sequence identity to SEQ ID NO: 4. Accordingly, the polypeptide for use in treatment and/or prevention of coronavirus infection according to the present invention has an amino acid sequence of at least about 60%, at least about 70%, at least about 80%, at least about 90%, at least about 95%, at least about 96%, at least about 97%, at least about 98%, at least about 99%, or 100% sequence identity to SEQ ID NO: 4.

In a second aspect, the present invention provides novel polypeptides, comprising an amino acid sequence selected from the group of SEQ ID NOs: 7, 6, 5, 8, preferably SEQ ID NO: 7, or an amino acid sequence of at least about 60% sequence identity to SEQ ID NOs: 7, 6, 5, 8. It is envisaged, that in the polypeptides of the second aspect of the present invention the substitutions (i) K26N, (ii) K26T, (iii) D14N, R17S, K26N, or (iv) D14N, R17S, K26T (relative to SEQ ID NO: 4) are constitutive substitutions and the remaining amino acid sequence may vary, while the amino acid sequence of the polypeptides of the present invention has at least about 60%, at least about 70%, at least about 80%, at least about 90%, at least about 95%, at least about 96%, at least about 97%, at least about 98%, at least about 99%, or 100% sequence identity to SEQ ID NOs: 5 to 8, respectively. Accordingly, a polypeptide of the second aspect of the present invention does not comprise or consist of SEQ ID NO: 4 or is not SEQ ID NO. 4.

In a third aspect, the present invention provides a polypeptide for use as a medicament, comprising an amino acid sequence according to SEQ ID NO: 4 or an amino acid sequence of at least 60% sequence identity to SEQ ID NO: 4. Accordingly, the polypeptide for use as a medicament according to the present invention has an amino acid sequence of at least about 60%, at least about 70%, at least about 80%, at least about 90%, at least about 95%, at least about 96%, at least about 97%, at least about 98%, at least about 99%, or 100% sequence identity to SEQ ID NO: 4.

The polypeptides to be used in the first, third aspect, and further aspects herein other than the second aspect may comprise the use of polypeptides according to the second aspect.

As used herein, the terms "comprising", "including", "having" or grammatical variants thereof are to be taken as specifying the stated features, integers, steps, or components but do not preclude the addition of one or more additional features, integers, steps, components, or groups thereof. The terms "comprising", "including", or "having" encompass the terms "consisting essentially of" and "consisting of", unless otherwise specifically indicated. Thus, whenever the terms "comprising", "including", or "having" are used herein, they can be replaced by "consisting essentially of" or, preferably, by "consisting of". The terms "comprising", "including", or "having" mean that any further component (or likewise features, integers, steps and the like) can be present. The term "consisting essentially of" or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed product, composition, use, method, and the like. The term "consisting of" means that no further component (or likewise features, integers, steps and the like) can be present.

The term "sequence identity", as used herein, refers to the sequence match between two polypeptides or nucleic acids. The polypeptide or nucleic acid sequences to be compared are aligned to give maximum identity, for example, using bioinformatics tools for pairwise alignment such as EMBOSS Needle (https://www.ebi.ac.uk/Tools/psa/emboss_needle/; see also Madeira et al. (2019). Nucleic Acids Research., 47, W1, W636-W641). When the same position in the sequences to be compared is occupied by the same nucleobase or amino acid residue, then the respective molecules are identical at that very position. Accordingly, the "sequence identity", "percent identity" or "percent sequence identity" is a function of the number of matching positions divided by the number of positions compared and multiplied by 100%. For example, if 6 out of 10 sequence positions are identical, then the identity is 60%. The "identity" or "percent (%) identity" between two amino acid sequences can, *e.g.,* be determined by using the Needleman-Wunsch algorithm (Needleman and Wunsch, (1970) J Mol Biol.,48, 3, 443-453*)* which has been incorporated into EMBOSS Needle, using a BLOSUM62 matrix, a "gap open penalty" of 10, a "gap extend penalty" of 0.5, a false "end gap penalty", an "end gap open penalty" of 10 and an "end gap extend penalty" of 0.5. The percent (%) identity is typically determined over the entire length of the query sequence on which the analysis is performed. Two molecules having the same primary amino acid or nucleic acid sequence are identical irrespective of any chemical and/or biological modification. For example, two polypeptides having the same primary amino acid sequence, but different glycosylation patterns are identical by this definition. In case of nucleic acids, for example, two molecules having the same sequence but different linkage components such as thiophosphate instead of phosphate are identical by this definition. It is known to the person skilled in the art that the term "polypeptide" refers to a biomolecule composed of a chain of amino acids linked together by peptide bonds. A "polypeptide" typically consists of 10 to 100 amino acids. In context of the present invention, the polypeptide may preferably comprise or consist of about thirty amino acids, for example, may comprise or consist of a contiguous amino acid sequence as shown in any one of SEQ ID NO: 4 to 8. In the context of the present invention, the sequence of the polypeptide may optionally include additional amino acid residues at its C-terminal and/or N-terminal end(s). These additional residues are preferably selected from the naturally occurring adjacent contiguous sequence of the RBD, examples of which are provided in SEQ ID NO: 1 (corresponding to the full-length S protein) or SEQ ID NO: 2 (corresponding to the RBD), and may extend the specific polypeptide sequence by up to 10 residues, such as 1, 2, 3, 4, 5, 6, 7, 8, 9, or 10 additional residue(s), at either or both termini. In other words, such additional residues are preferably contiguous sequences of between 1 to 10 additional amino acid residues corresponding to the sequence that is present N-terminally and/or C-terminally to, e.g., SEQ ID NOs 4, 5, 6, 7 or 8 in the RBD, e.g., as shown SEQ ID NO: 1 or SEQ ID NO: 2. For example, such N-terminal amino acid residues may be a contiguous stretch of one or more amino acids of VSPTKLNDLC (e.g., C, LC, DLC, NDLC, LNDLC, KLNDLC, TKLNDLC, PTKLNDLC, SPTKLNDLC or VSPTKLNDLC and/or such C-terminal amino acid residues may be a contiguous stretch of one or more amino acids of NYKLPDDFTG (e.g., G, TG, FTG, DFTG, DDFTG, PDDFTG, PDDFTG, LPDDFTG, KLPDDFTG, YKLPDDFTG or NYKLPDDFTG). Such extensions may, *inter alia,* contribute to structural stability, improved expression, or enhanced biological activity. However, only those extensions are considered suitable that preserve the function of the polypeptide, as defined in the present invention.

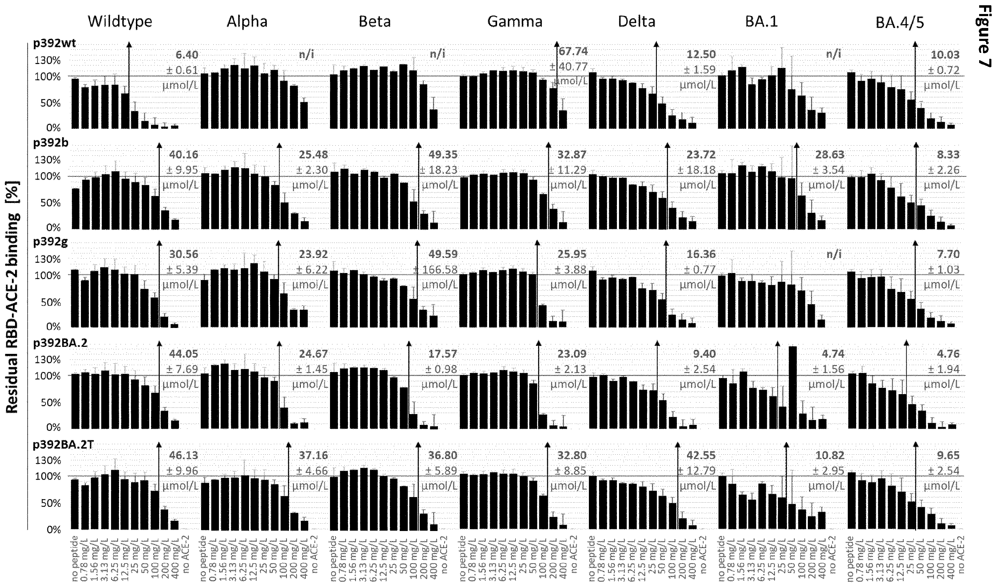

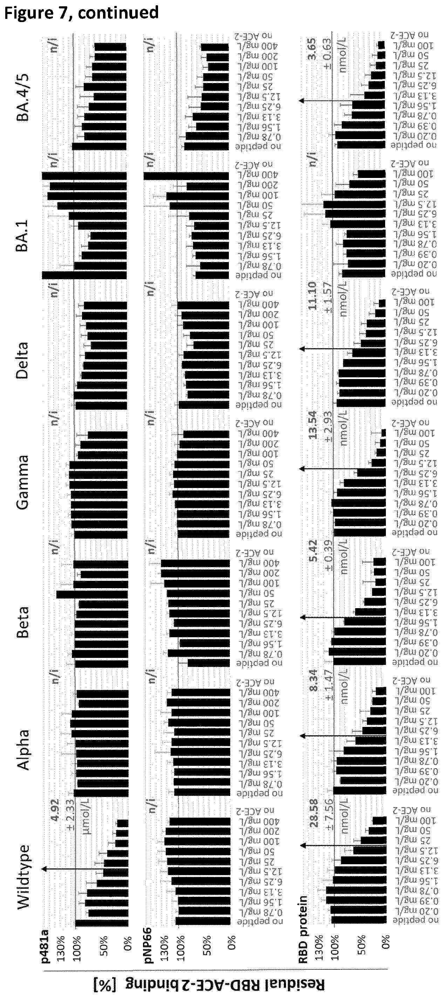

In context of the present invention, and as illustrated in the appended non-limiting examples provided herein, the polypeptides of the present invention are derived from the N-terminal region of the RBD of the SARS-CoV-2. More specifically, from a region spanning the amino acid residues 392 to 421, in relation to the full-length S protein (SEQ ID NO: 1). The polypeptides of the present invention are derived from the amino acid sequence of the ancestral strain (e.g., SEQ ID NO: 4) or may resemble residues derived from, but are not limited to, important SARS-CoV-2 VOCs, such as alpha, beta, gamma, delta (kappa), and omicron variants BA.1 to BA.5, BQ.1, XBB, and KP.3 (e.g., SEQ ID NOs: 5-8). Accordingly, the polypeptides of the present invention may comprise one or more amino acid substitutions at amino acid residues, preferably selected from the group of D405, R408, and K417 and combinations thereof, relative to the full-length S protein (SEQ ID NO: 1). Examples of preferable amino acid substitutions and combinations thereof include, but are not limited to, (i) K417N, (ii) K417T, (iii) D405N, R408S, and K417N, or (iv) D405N, R408S, and K417T. In context of the present invention, amino acid residues D405, R408, and K417 relative to the full-length S protein (SEQ ID NO: 1) are equivalent to amino acid residues D14, R17, and K26 relative to the SEQ ID NO: 4. Accordingly, the present invention further provides a polypeptide comprising amino acid substitutions relative to SEQ ID NO: 4 selected from: (i) K26N, (ii) K26T, (iii) D14N, R17S, K26N, or (iv) D14N, R17S, K26T. Accordingly, polypeptides comprising amino acid substitutions relative to SEQ ID NO: 4 (i) K26N, (ii) K26T, (iii) D14N, R17S, K26N, or (iv) D14N, R17S, K26T, (such as SEQ ID NO. 5, 6, 7, and 8) are preferably to be used in the prevention and/or treatment of SARS-CoV-2 VOCs, such as alpha, beta, gamma, delta (kappa), and omicron variants BA.1 to BA.5, XBB, and KP.3. As shown by the non-limiting example, particular polypeptides of the present invention may be more advantageous for blocking complex formation between a surface protein of a specific coronavirus variant and a receptor of a target cell than others (see, e.g., **Example 4** and **Figures 7****).** The person skilled in the art is capable of determining which specific polypeptide of the present invention is to be used to block complex formation and/or treat and/or prevent coronavirus infection. Methods to determine the inhibitory effect on the complex formation are described herein, specific examples are illustrated in the non-limiting examples provided herein. In particular, polypeptides comprising amino acid substitutions relative to SEQ ID NO: 4 (i) K26N, (ii) K26T, (iii) D14N, R17S, K26N, or (iv) D14N, R17S, K26T, respectively, (such as SEQ ID NO. 5, 6, 7, and 8, respectively) are preferably to be used in, but are not limited to, the prevention and/or treatment of SARS-CoV-2 VOCs, such as omicron variant BA.4, omicron variant BA.5, or alpha, omicron variant BA.4, omicron variant BA.5, delta, or alpha, omicron variant BA.4, omicron variant BA.5, delta, beta, or gamma, omicron variant BA.4, or omicron variant BA.5, respectively. In line with this, preferred but non-limiting examples of the amino acid sequence of the polypeptides of the present invention may be selected from SEQ ID NOs: 7, 6, 5, and 8. A preferred example for the amino acid sequence of a polypeptide of the present invention is SEQ ID NO: 7. Accordingly, preferred polypeptides of the present invention may be selected from the polypeptides consisting of SEQ ID NOs: 7, 6, 5, and 8, preferable herein is the polypeptide consisting of SEQ ID NO: 7.

The terms "amino acid" and "amino acid residue" may be used synonymously herein. The terms "substituted", "replaced" and "mutated" may be used synonymously herein. Accordingly, also the terms "substitution", "replacement" and "mutation" may be used synonymously herein. An example for a substitution would be that the lysine (K) at position 26 in SEQ ID NO: 4 or at a position corresponding to this position is substituted by asparagine (N). Another example for a substitution would be that the lysine (K) at position 26 in SEQ ID NO: 4 or at a position corresponding to this position is substituted by threonine (T). The skilled person is well aware how amino acids can be substituted by molecular biological techniques; a non-limiting example thereof is site-directed mutagenesis.

As illustrated in the appended non-limiting examples provided herein, the polypeptides of the present invention may be further modified at their N- and/or C-terminus. Such modifications are known in the art. In context of the present invention, N-terminal modifications are not particularly limited but a non-limiting example thereof may be an amino group of the amino acid X₁ or a modification of the N-terminal amino group with the general formula NR1R2, whereas R1 and R2 are independent from each other preferably selected from hydrogen or the groups consisting of: (i) a straight chain, branched, cyclic, or heterocyclic alkyl group, such as methyl, ethyl, n-propyl, isopropyl, n-butyl, isobutyl, cyclohexyl; (ii) a straight chain, branched, cyclic or heterocyclic alkanoyl group, such as acetyl or methanoyl (formyl), propionyl, n-butyryl, isobutyryl, pentanoyl, hexanoyl, or cyclohexanoyl; (iii) a reporter group, such as a fluorescent dye (e.g. fluorescein, Alexa488) or biotin; (iv) a linker bridging the N- and C-termini thereof to obtain a cyclic peptide. Preferable herein, the N-terminal modification may be an N-terminal acetylation. In context of the present invention, C-terminal modifications are not particularly limited but a non-limiting example thereof may be a free C-terminal carboxyl group of the C-terminal amino acid or a modification of the C-terminal carboxyl group, preferably with the general formula COR3 (R3 replacing the hydroxyl group of the last amino acid), wherein COR3 is preferably selected from the group consisting of: (i) carboxyl (R3 being a free hydroxyl), an ester (R3 being alkoxy), an amide (R3 being an amine) or an imide; (ii) a linker bridging the N- and C-termini thereof to obtain a cyclic peptide. Preferable herein, the C-terminal modification may be a C-terminal amidation. Accordingly, the polypeptides of the present invention may preferably be N-terminally acetylated and/or C-terminally amidated. A particularly preferred modification of the polypeptides of the present invention is C-terminal amidation.

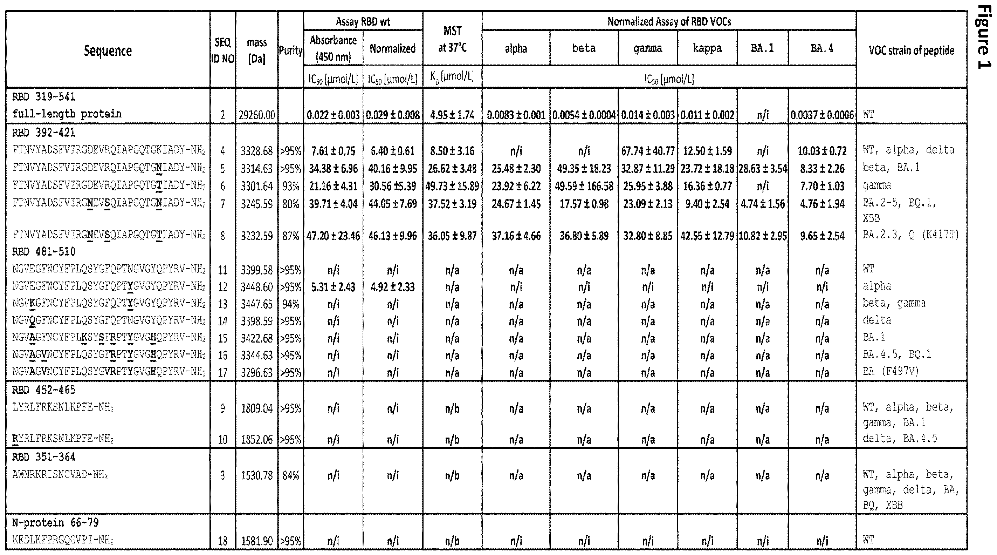

As illustrated in the appended non-limiting examples provided herein, the inventors have investigated four polypeptide families derived from different RBD regions (corresponding to residues 351 to 364, 392 to 421, 452 to 465, and 481 to 510 of the S protein sequence) of wild-type SARS-CoV-2 and six relevant VOCs containing previously reported interaction sites in complex with ACE-2 for their inhibitory effects (see, e.g., **Example 2** and **Figure 1****).** The ACE-2 binding affinity of the polypeptides and their inhibitory effect on RBD-ACE-2 complex formation (IC₅₀) correlated well with inhibition of HEK293 cell infection in a pseudovirus assay. Surprisingly, the most promising polypeptides were of the p392 family, i.e. derived from the N-terminal region of the RBD of the SARS-CoV-2, more specifically from the residues 392 to 421 of the S protein of the ancestral strain (SEQ ID NO: 4) and of relevant VOCs (SEQ ID NOs: 5 to 8). The polypeptide p392BA., corresponding to residues 392 to 421 of the S protein of VOC omicron BA.2 (mutations D405N, R408S, K417N of the wild-type strain2; see SEQ ID NO: 7), was the most promising as it inhibited ACE-2 binding of all VOC RBDs in the low micromolar range. Although the pseudovirus assay was limited to the wild-type S protein and did not include other VOCs, the p392 sequence appears to be highly conserved up to the most recent BA.2 variant and thus should inhibit other VOCs, as indicated by the *in vitro* assay.

The polypeptides of the present invention have particularly high specificity to the target cell receptor and exhibit strong binding thereto. As illustrated in the appended non-limiting examples provided herein, it could convincingly be shown that the polypeptides of the present invention show particularly strong binding to the ACE-2 receptor (see, e.g., **Example 3,** **Figures 1 to 2****).** Accordingly, the present invention provides that the polypeptides of the present invention bind to a receptor of a target cell, preferably wherein the receptor of a target cell is a membrane receptor of a target cell, more preferably wherein the membrane receptor of a target cell is an ACE-2 receptor.

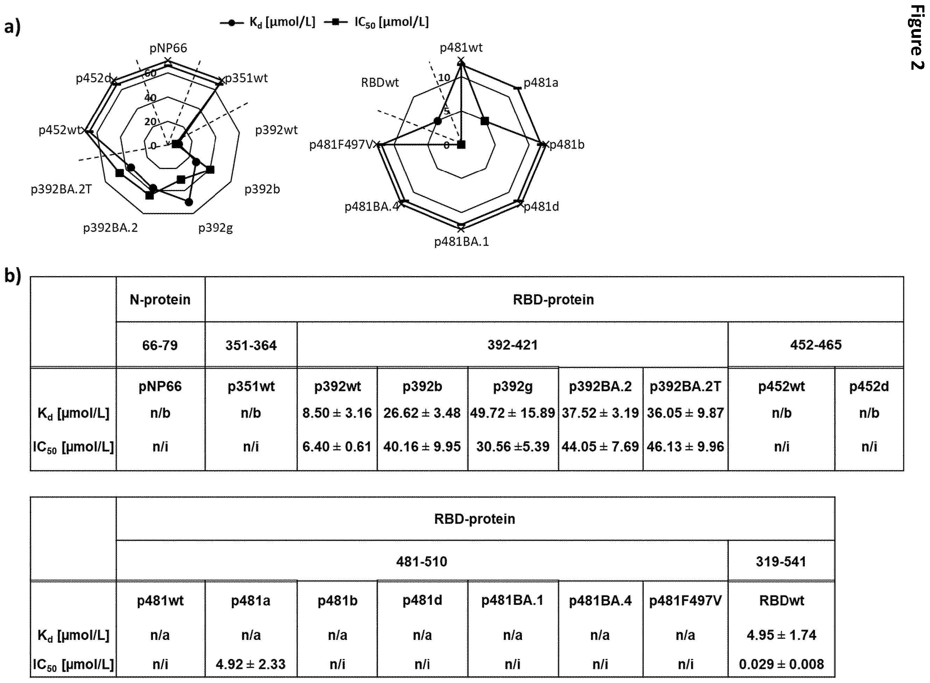

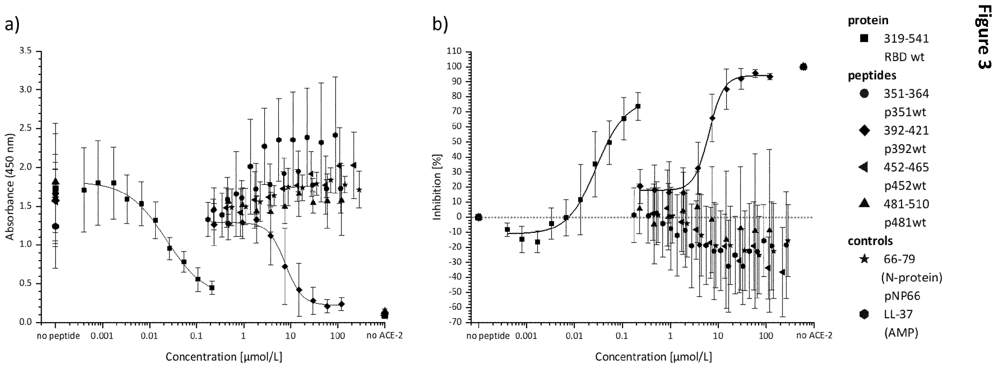

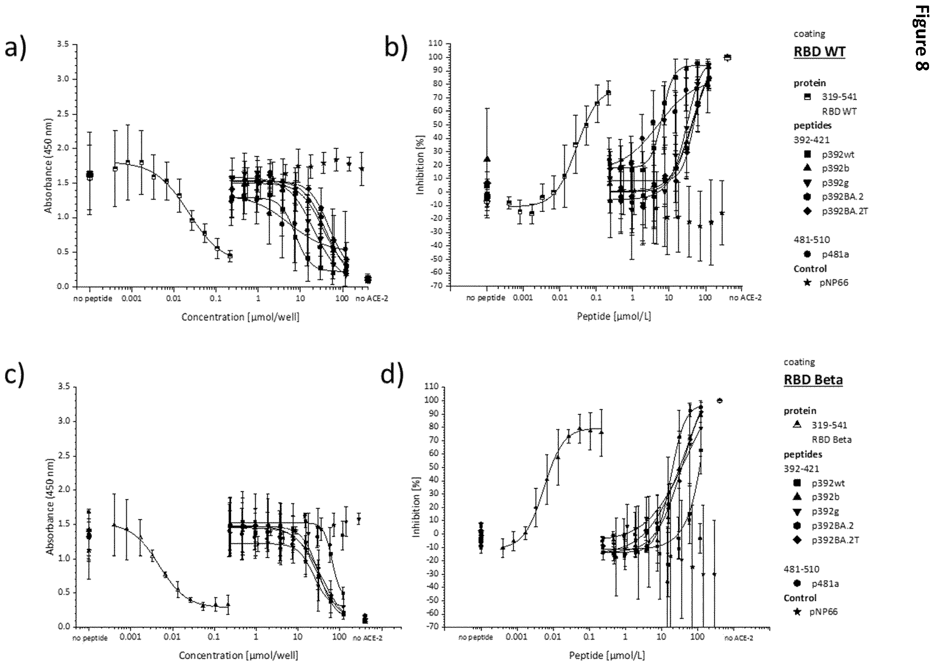

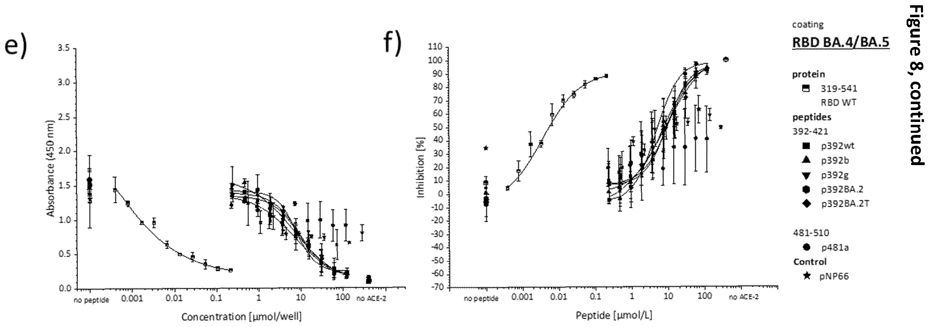

As discussed herein above and as also illustrated in the appended non-limiting examples provided herein, the inventors have shown, by employing an established inhibition assay (see e.g., **Example 1-E** or Schwarze et al. (2022)) and Microscale thermophoresis (MST; see, e.g., **Example 1-F,** NanoTemper Technologies. Inc. (2019), or Sadremomtaz et al. (2022)), that the polypeptides of the present invention exhibit good inhibition of the RBD-ACE-2 receptor binding. Using an RBD-ACE-2 binding assay with the recombinant RBD coated on a microtiter plate, the inventors unexpectedly observed very good inhibition of up to 96% (see, for example, **Example 3** and **Figures 3 to 4****).** This inhibition was shown not only for wildtype RBD (of the ancestral SARS-CoV-2 strain (SEQ ID NO: 2; see, e.g., **Example 3** and **Figures 2 to 5****)** but also for the RBD of six relevant VOCs (see, e.g., **Example 4** and **Figures 8 to 10****).** The polypeptide p392BA.2 (SEQ ID NO: 7), which resembles residues 392 to 421 of the omicron variants BA.2-5, BQ.1, XBB, and KP.3 showed the strongest effect against all VOCs tested with an average IC₅₀ of 18.33 µmol/L (see, e.g., **Example 4** and **Figures 7 to 10****).** Interestingly, this sequence is conserved in the omicron BA.2 variant and may therefore have an inhibitory effect against more recent VOCs including XBB, KP.3, and future SARS-CoV-2 variants. Accordingly, the present invention provides that the polypeptides of the present invention prevent target cell penetration of the coronavirus. The present invention further provides that the polypeptides of the present invention block the complex formation between a surface protein of the coronavirus and receptor of the target cell. It is particularly envisaged that by blocking said complex formation the polypeptides of the present invention prevent target cell penetration of the coronavirus.

As the polypeptides of the present invention inhibit the first step of the cell infection cycle, some aspects of the present invention relate to the prophylactic (i.e., preventive) use of the polypeptides of the present invention. In context of the present invention, the term "prevention" (and grammatical variations thereof such as "prevent" or "preventing") relates to obtaining a prophylactic benefit in a subject. With respect to achieving a prophylactic benefit, the object is to delay or prevent the symptoms or underlying cause (i.e., viral infection) associated with a coronavirus infection. Preferably, the object is to prevent coronavirus infection without causing physiologically negative consequences for the subject. Desirable effects of prevention include, but are not limited to, prophylaxis and preventing occurrence or recurrence of disease or symptoms associated with disease.

A further gist of the present invention is that the polypeptides of the present invention can be used to treat an existing coronavirus infection. In context of the present invention, the terms "treatment" (and grammatical variations thereof such as "treat" or "treating") relates to obtaining a therapeutic benefit in a subject. With respect to achieving a therapeutic benefit, the object is to eliminate, lessen, decrease the severity of, ameliorate, or slow the progression of the symptoms or underlying cause (i.e., viral infection) associated with a coronavirus infection. Desirable effects of treatment include, but are not limited to, alleviation of symptoms, diminishment of any direct or indirect pathological consequences of the disease, decreasing the rate of disease progression, amelioration or palliation of the disease state, improved prognosis, and cure.

In context of the present invention, the "subject" or "individual" to be treated or in which the coronavirus infection is to be prevented may also herein referred to as the "patient". Furthermore, one or more cell(s) within or derived from a subject's body that may be subject to a coronavirus infection is herein referred to as "target cell(s)". The subject may be any animal susceptible to coronavirus infection. In particular, examples for the subject include, but are not limited to, mammals such as humans, bats, swine, cattle, horses, camels, cats, dogs, and rodents, or birds. Preferably herein the subject is a human. The state of health of the subject is not particularly limited, and the subject may or may not have had a prior coronavirus infection.

The present invention envisions that the polypeptides of the present invention are administered in a therapeutically effective amount. The term "effective amount" refers to an amount sufficient to induce a detectable therapeutic response in the subject to which the polypeptides or pharmaceutical composition is to be administered.

As laid out herein above, the present invention is particularly advantageous for individuals with compromised or suppressed immune systems. Accordingly, the polypeptides of the present invention may be used for treating and/or preventing of coronavirus infection wherein the subject in need therefore has a compromised or suppressed immune system.

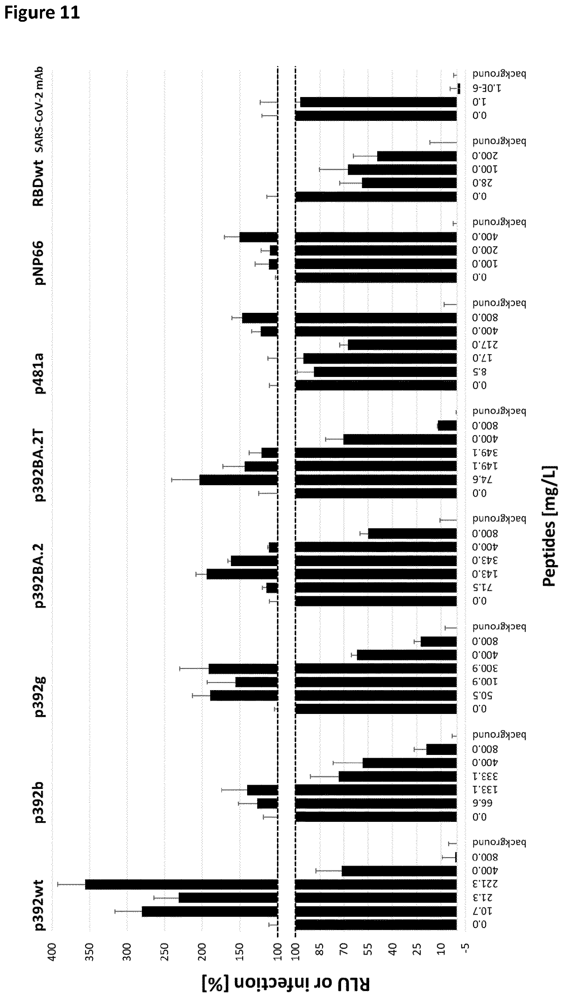

As discussed herein above and as illustrated in the appended non-limiting examples provided herein, the polypeptides of the present invention effectively inhibited ACE-2 receptor binding to the corresponding coated RBD variant with IC₅₀ values ranging from 4.74 ± 1.56 µmol/L (p392BA.2 vs. omicron BA.1) to 67.74 ± 40.77 µmol/L (p392wt vs. gamma). The tested p392 polypeptides showed an inhibition of 50% already at concentrations of ≥ 5 µmol/L, and for some of those polypeptides an inhibition of 90% occurred at just ≥ 15 µmol/L (see, e.g., **Figure 7****).** Accordingly, a polypeptide of the present invention may have IC₅₀ concentration of at least 85 µmol/L, preferably at least 80 µmol/L, more preferably at least 70 µmol/L, more preferably at least 60 µmol/L, more preferably at least 50 µmol/L, more preferably at least 40 µmol/L, more preferably at least 30 µmol/L, more preferably at least 20 µmol/L, more preferably at least 15 µmol/L, more preferably at least 10 µmol/L, more preferably at least 9 µmol/L, more preferably at least 8 µmol/L, more preferably at least 7 µmol/L, more preferably at least 6 µmol/L, most preferably at least 5 µmol/L, using the respective coronavirus variant RBD as a competitor for target cell receptor binding in the RBD-ACE2 assay as described herein. The *in vitro* inhibitory effect of the RBD-ACE2 assay which showed that all investigated p392 polypeptides evaluated demonstrated good inhibition, in particular against omicron variant BA.4/5, has also been confirmed by an *in vitro* pseudovirus assay using HEK293 cells expressing ACE-2 (see, e.g., **Example 5** and **Figure 11****).** Almost all tested p392 polypeptides reduced the pseudovirus infection rate to a range of 58% to 71% at a polypeptide concentration of 400 mg/L, equivalent to 122 µM, and at a polypeptide concentration of 800 mg/L, equivalent to 244 µM, all p392 polypeptides reduced the viral infection rate further to a range of 1.2% to 55% which translates to infection inhibition rates of 45% to 99.8%. As evident from the appended non-limiting examples preferable inhibition (%) of the polypeptides of the present invention, when determined by the herein described pseudovirus test (see. e.g., Example 1-H), may be between about 30% and 100%, preferably at least about 30%, at least 40%, at least about 50%, at least about 60 %, at least about 70%, at least about 80%, at least about 85%, at least about 90% at least about 95%, at least about at least about 96%, at least about 97%, at least about 98%, at least about 99%, or 100%. As indicated in the appended non-limiting examples, the herein described pseudovirus assay uses cells that show an overexpression of ACE-2 receptor (see. e.g., Example 1-H). Accordingly, those cells present many more ACE-2 receptors on the surface as comparable *in vivo target* cells (e.g., human epithelial cells of the respiratory tract). Accordingly, it is envisaged that the treatment and/prevention of coronavirus infection *in vivo* is much more effective than indicated by the presented pseudovirus (see, e.g., **Example 5,** **Figure 11****).** Furthermore, as illustrated in the appended non-limiting examples, the herein described pseudovirus only used the wild-type RBD sequence (see, e.g. SEQ ID NO: 2). In the case of the wild-type RBD pseudovirus assay, p392BA.2 is less effective than p392wt in inhibiting coronaviral infection (see, e.g., **Figure 11****),** which is consistent with the data from the RBD-ACE2 assay also provided herein (see, e.g., **Figure 7****).** Accordingly, it is expected that, for example, the exemplary non-limiting p392BA.2 polypeptide (see SEQ ID NO: 7) would perform as excellent in a pseudovirus assay, using the omicron variant BA.2 RBD sequence, as indicated by the results obtained in the ACE2-RBD assay using the omicron variant BA.4/5 RBD sequence (see, e.g., **Figure 7****).** Accordingly, it is envisaged that the effectiveness of the polypeptide is preferably determined by the determination of the IC₅₀ concentration, preferably by the methods described herein. However, the person skilled in the art is aware that the effective inhibition of a polypeptide may optionally be determined by other means and methods, some of which are also further described herein. Accordingly, a preferable polypeptide would be a polypeptide comprising an amino acid sequence selected from the group of SEQ ID NOs: 7, 6, 5, 8, 4, most preferably SEQ ID NO: 7, or an amino acid sequence of at least 80% sequence identity to SEQ ID NOs: 7, 6, 5, 8, 4 (see. e.g. **Figure 7****)** as, at the present time, mostly omicron variants still in circulation comprise the sequence SEQ ID NO: 7. As laid out herein, the person skilled in the art is thus capable to select a polypeptide in accordance with the present invention based on the currently most active coronavirus variants.

The person skilled in the art is aware that inhibitory effects of an agent, such as a polypeptide of the present invention, can be determined, for example *in vitro* or *ex vivo,* illustrative examples are provided herein in the appended non-limiting examples. Methods for determining inhibitory effect of an agent are known in the art. A non-limiting example for an *in vitro* method for measuring the inhibitory effect of a polypeptide may be as described by Schwarze and colleagues (Schwarze et al. (2022). Frontiers in immunology, 13, S. 915034), such as incubating microplates coated with RBD protein (for example SARS-CoV-2 RBD protein) with a solution containing ACE-2 and the polypeptides to be tested, determining the binding of the ACE-2 and RBD protein, for example by densitometric means, such as OD₄₅₀ measurement. Control samples would be incubating microplates coated with RBD protein with a solution either without polypeptide (negative control, 0% inhibition) or without ACE-2 (positive control, 100% inhibition). To determine and normalize the residual binding (%) and Inhibition (%) the following exemplary but non-limiting calculation may be used: Residual binding (%) = 100 x ((sample OD₄₅₀-positive control OD₄₅₀) / (negative control OD₄₅₀ - positive control OD₄₅₀)); inhibition (%) = 100 - residual binding (%); from mg/L to µmol/L: (X mg/L / X Da (monoisotopic polypeptide mass)) / 1000 = X µmol/L (see also, e.g., non-limiting **Example 1-E** or Schwarze et al. (2022)). A non-limiting example for an *ex vivo* or *in vitro* method for measuring the inhibitory effect of a polypeptide may be seeding a fixed number of target cells (e.g., HEK293) of a tissue culture and incubating the culture with a relevant virus (e.g., a SARS-CoV-2 pseudovirus). After a set period of incubation (e.g., 45 minutes and/or 90 minutes) the infection rate of the tested virus is evaluated (by methods known in the art). Such measuring the inhibitory effect of a polypeptide may be conducted with commercially available test kit, a non-limiting example of such a kit would be the "COVID-19 Pseudovirus Neutralizing Antibody Assay (Luciferase)" from Abnova Corporation (Catalog Number KA6152); see also, e.g., non-limiting **Example 1-H.**

In context of the present invention, an "agent" refers to any substance, compound, composition, molecule, or biological entity that performs a specified function, induces a desired effect, or interacts with a target in a composition, formulation, or process. The agent may include, but is not limited to, chemical compounds, pharmaceuticals, biologics, antibodies, enzymes, nucleic acids, small molecules, or any combination thereof.

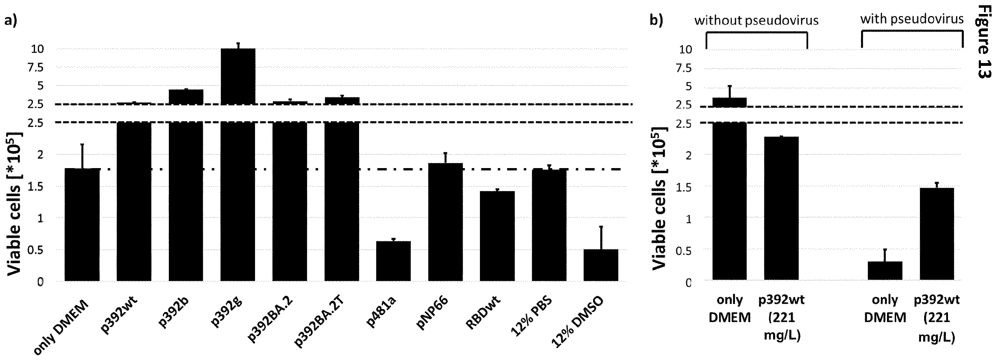

As illustrated in the appended non-limiting examples provided herein, it could convincingly be shown that the polypeptides of the present invention exhibit no cytotoxic effect, even at concentrations of 800 mg/L (see, e.g., **Example 6** and **Figure 13****).** Accordingly, the present invention provides a polypeptide for use in prevention and/or treatment of coronavirus infection, wherein the therapeutically effective amount is in the range of about 0.33 mg/L to about 33 g/L, equivalent to about 100 nM to about 10 mM, preferably of about 1.65 mg/L to about 3.3 g/L, equivalent to about 500 nM to about 1 mM, more preferably of about 3.3 mg/L to about 1.65 g/L, equivalent to about 1 to about 500 µM, most preferably of about 33 to about 800 mg/L, equivalent to about 10 to about 244 µM. Accordingly, the present invention most preferably provides a polypeptide of the present invention for use in prevention and/or treatment of coronavirus infection, wherein the therapeutically effective amount is in the range of about 33 to about 800 mg/L, equivalent to about 10 to about 244 µM. It is evident for the skilled person that also higher or lower concentrations than between about 10 to about 244 µM can be employed without deviating from the gist of the present invention. However, it has been shown herein and is illustrated in the non-limiting appended examples that the polypeptides of the present invention can successfully be employed in the treatment and/or prevention of coronavirus infection, preferably at concentrations between about 10 to about 244 µM. It is envisaged that the dosage range of the therapeutically effective amount of the polypeptides of the present invention will vary depending on the route of application. Exemplary, but non-limiting, dosage for systemically administration, such as subcutaneously injection, may be between about 1 to about 50 mg/kg bodyweight, preferably between about 5 to about 40 mg/kg, more preferably between about 10 to about 30 mg/kg. A further exemplary, but non-limiting, dosage for administration by inhalation, such as aerosol inhalation, may be between about 0.1 to about 10 mg/kg bodyweight, preferably between about 0.5 to about 5 mg/kg bodyweight, more preferably between about 1 to about 2 mg/kg bodyweight.

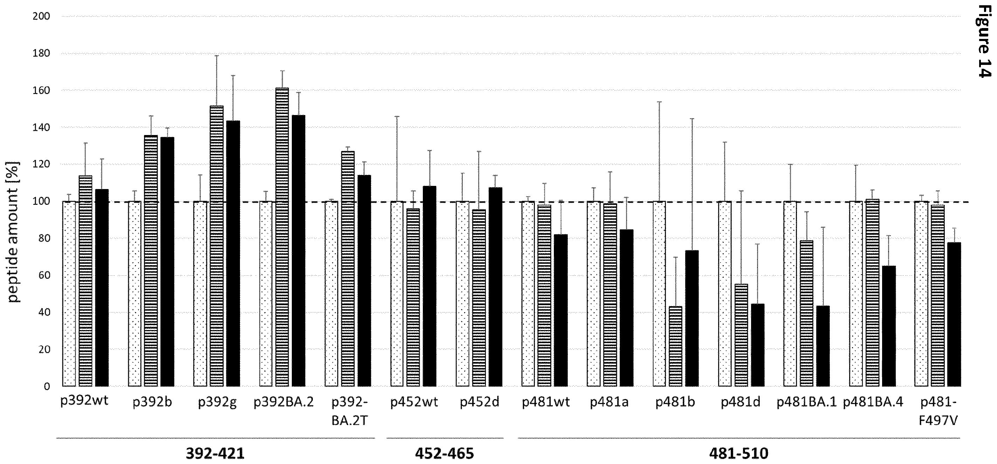

As illustrated in the appended non-limiting examples provided herein, it could convincingly be shown that the protease stability of the polypeptides of the present invention is exceptional (see, e.g., **Example 7** and **Figure 14****).** The person skilled in the art is aware that a coronavirus infection primarily affects the respiratory system, in particular the upper respiratory tract and/or the lower respiratory tract. Examples for the upper respiratory tract include, but are not limited to, the ciliated epithelium of the nasopharynx, sinuses, nose, and throat. Examples for the lower respiratory tract include, but are not limited to, the windpipe and lungs, particularly the alveoli (air sacs). Accordingly, the polypeptides of the present invention may be administered parenterally or orally. Examples for a parenteral administration include, but are not limited to, a spray, such as a nasal or oral spray, inhalation, such as vapor, powder, or aerosol inhalation, or injection. Preferably parenteral administration is by nasal spray or aerosol inhalation.

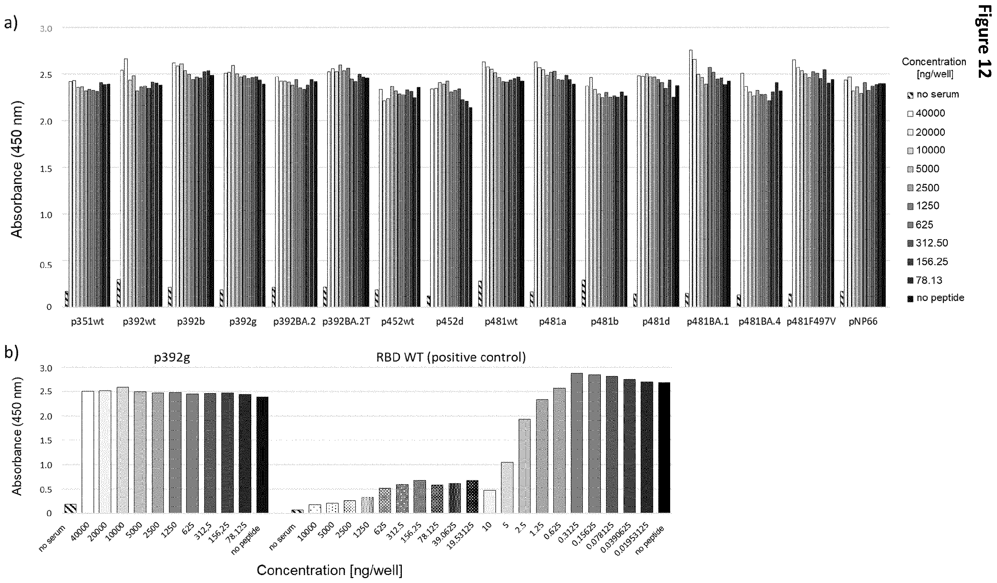

Furthermore, it is envisaged that the polypeptides of the present invention may be administered in combination with one or more further antiviral agent(s). In context of the present invention, the further antiviral agent is not particularly limited, and examples thereof may be mRNA vaccines, adenovirus vector vaccines, inactivated virus vaccines, subunit vaccines, monoclonal antibody therapies, or other small molecule treatments. Preferably, the one or more further antiviral agent(s) is selected from the group of Remdesivir, Nirmatrelvir, Ritonavir, Dexamethasone, and Tocilizumab. As illustrated in the appended non-limiting examples provided herein, the inventors have shown that the polypeptides of the present invention are not recognized by anti-S1 antibodies (i.e., antibodies that specifically bind to the S1 subunit of the spike (S) protein of a coronavirus. The S1 subunit is part of the S protein and contains the RBD.) induced by vaccination, such as, e.g., mRNA vaccines from Moderna (mRNA-1273) or BioNTech (BNT162b2) (see, e.g., **Example 8** and **Figure 12****).** Accordingly, the polypeptides of the present invention can be also used in combination with vaccination strategies to prevent and/or treat coronavirus infection and thereby creating a synergistical treatment effect. In line with this, the further antiviral agent may be an antibody, preferably wherein the antibody binds a coronavirus, more preferably wherein the antibody binds the spike (S) protein of the coronavirus, even more preferably wherein the antibody binds the RBD of the spike (S) protein. In context of the present invention, the coronavirus bound by an antibody as a further antiviral agent may be selected from the group of SARS-CoV-2, SARS-CoV-1, HCoV-NL63, and related Coronaviridae, preferably SARS-CoV-2. Alternatively, the antibody administered as a further antiviral agent may bind the ACE-2 receptor.

In context of the present invention antibodies that bind to a coronavirus or the ACE-2 receptor may be monoclonal, polyclonal, recombinant, or engineered antibodies, including full-length immunoglobulins, antigen-binding fragments (e.g., Fab, F(ab')₂, scFv), nanobodies, or antibody-drug conjugates. Such antibodies may be intended for use in diagnostic, prophylactic, or therapeutic applications, including virus neutralization, inhibition of viral entry, or detection of coronavirus infection.

In another aspect, the present invention provides the use of the polypeptides of the present invention for potentiating the antiviral effect of one or more agent(s), wherein the one or more agents(s) is/are selected from the group of mRNA vaccines, adenovirus vector vaccines, inactivated virus vaccines, subunit vaccines, monoclonal antibody therapies or other small molecule treatments. Preferably herein, the one or more agent(s) is/are selected from the group of Remdesivir, Nirmatrelvir, Ritonavir, Dexamethasone, and Tocilizumab. The one or more agent(s) having an antiviral effect may also be an antibody, preferably wherein the antibody binds a coronavirus, more preferably wherein the antibody binds the spike (S) protein of the coronavirus, even more preferably wherein the antibody binds the RBD of the spike (S) protein. In context of the present invention, the coronavirus bound by an antibody as a further agent having antiviral effect may be selected from the group of SARS-CoV-2, SARS-CoV-1, HCoV-NL63, and related Coronaviridae, preferably SARS-CoV-2. Alternatively, the antibody administered as a further agent having antiviral effect may bind the ACE-2 receptor. In other words, the polypeptides of the present invention may be used to achieve synergistical effect with other antiviral treatment options.

In context of the present invention, the polypeptide and the one or more further antiviral agent(s) may be administered simultaneously or sequentially during the course of the same treatment period. In context of the present invention, the administration frequency is not particularly limited, but examples thereof are once or multiple times during the same treatment period. Examples for multiple administrations may be two, three, four, or at least five times. Administration frequency can also naturally depend on the form of administration. The administration frequency can be, for example, three times a day, twice a day, once a day, once in two days, once in three days, once in four days, once in five days, once in six days, once in a week, once in ten days, or once in two weeks. The administration frequency can also include dosing holidays, for example, from about 1 day to about 7 days between administration. However, as illustrated in the appended examples a single administration may be enough to treat and/or prevent infection, see illustratively in appended **Example 5.**

In a further aspect, the present invention relates to a composition comprising a polypeptide of the present invention. Thus, the invention provides a composition comprising a polypeptide of the present invention as described herein above.

Furthermore, the present invention relates to a composition as described herein for use as a medicament. Thus, the present invention provides a composition for use as a medicament comprising a polypeptide of the present invention, as defined herein above, as an active ingredient.

The present invention also relates to a composition as described herein for use in treatment and/or prevention of coronavirus infection. Thus, the present invention provides a composition for use in treatment and/or prevention of coronavirus infection comprising a polypeptide of the present invention, as defined herein above, as an active ingredient.

In a further aspect, the present invention relates to a pharmaceutical composition comprising a polypeptide of the present invention and a pharmaceutical acceptable carrier, excipient, or diluent. Thus, the invention provides a pharmaceutical composition comprising a polypeptide of the present invention, as defined herein above, and at least one pharmaceutical acceptable carrier, excipient, and/or diluent.

Furthermore, the present invention relates to a pharmaceutical composition as described herein for use as a medicament. Thus, the present invention provides a pharmaceutical composition for use as a medicament comprising a polypeptide of the present invention, as defined herein above, and at least one pharmaceutical acceptable carrier, excipient, and/or diluent.

The present invention also relates to a pharmaceutical composition as described herein for use in treatment and/or prevention of coronavirus infection. Thus, the present invention provides a pharmaceutical composition for use in treatment and/or prevention of coronavirus infection comprising a polypeptide of the present invention, as defined herein above, and at least one pharmaceutical acceptable carrier, excipient, and/or diluent.

In context of the present invention, the term "pharmaceutical composition" may be used interchangeably herein with the term "drug".

The pharmaceutical composition, in accordance with the present invention, may include in addition to a polypeptide of the present invention at least one (e.g., one or more selected from the group of) pharmaceutically acceptable carrier, excipients, auxiliary substances, preservatives, solvents, pH adjusting agents, and/or viscosity modulating agents. The person skilled in the art knows suitable formulations for respective pharmaceutical compositions and will readily be able to choose suitable compounds, such as pharmaceutically acceptable carriers or excipients, depending, e.g., on the formulation and/or administration route of the pharmaceutical composition of the invention. The pharmaceutically acceptable carrier is not particularly limited, and non-limiting examples thereof may include glucose, lactose, sucrose, starch, mannitol, dextrin, fatty acid glycerides, polyethylene glycol, hydroxyethyl starch, ethylene glycol, polyoxyethylene sorbitan fatty acid esters, amino acids, gelatine, albumin, water, a physiological saline solution and the like. According to the need, a generally used additive such as a stabilizer, a wetting agent, an emulsifier, a binder, a tonicity agent, a permeant, and/or an excipient can be suitably added. Non-limiting examples of a solvent include water, saline solution or any other physiological solution, ethanol, DMSO, glycerol, oil such as vegetable oil, or a mixture thereof.

The administration frequency of the composition and/or the pharmaceutical composition of the present invention is not particularly limited, but an example thereof is once or multiple times during the same treatment period. Examples for multiple administrations may be two, three, four, or five times. Administration frequency can also naturally depend on the form of administration. The administration frequency can be, for example, three times a day, twice a day, once a day, once in two days, once in three days, once in four days, once in five days, once in six days, once in a week, once in ten days, or once in two weeks. The administration frequency can also include dosing holidays, for example, from about 1 day to about 7 days between administration. However, as illustrated in the appended examples a single administration may be enough to treat and/or prevent infection.

The invention also relates to nucleic acid molecules encoding the herein described polypeptides. Thus, the present invention provides a nucleic acid molecule encoding a polypeptide of the invention. In context of the present invention, the nucleic acid molecule may be an DNA molecule or an RNA molecule encoding a polypeptide of the invention. The present invention relates to a nucleic acid molecule encoding an amino acid sequence according to SEQ ID NO: 4 or an amino acid sequence of at least 80% sequence identity to SEQ ID NO: 4. The present invention further relates to a nucleic acid molecule encoding an amino acid sequence comprising the amino acid substitutions relative to SEQ ID NO: 4 selected from (i) K26N, (ii) K26T, (iii) D14N, R17S, K26N, or (iv) D14N, R17S, K26T. Preferably, the present invention relates to a nucleic acid molecule encoding an amino acid sequence selected from the group of SEQ ID NOs: 7, 6, 5, and 8, preferably SEQ ID NO: 7.

The present invention further relates to a vector comprising the herein described nucleic acid molecules. Thus, the present invention provides a vector comprising a nucleic acid molecule encoding a polypeptide of the invention. Vectors that can be used in accordance with the present invention are known in the art. The vectors can further comprise expression control sequences operably linked to the nucleic acid molecules of the present invention contained in the vectors. These expression control sequences may be suited to ensure transcription and synthesis of a translatable RNA, *in vivo* or *in vitro.* Expression control sequences can for instance be promoters. Promoters for use in connection with the nucleic acid molecules of the present invention may be homologous or heterologous with regard to its origin and/or with regard to the gene to be expressed. Suitable promoters are for instance promoters which lend themselves to constitutive expression. However, promoters which are only activated at a point in time determined by external influences can also be used. Artificial and/or chemically inducible promoters may be used in this context. Preferably, the vector of the present invention is an expression vector. Expression vectors have been widely described in the literature; a non-limiting example may be the pHLsec expression vector. As a rule, expression vectors contain not only a selection marker gene and a replication-origin ensuring replication in the host selected, but also a bacterial or viral promoter, and in most cases a termination signal for transcription. Between the promoter and the termination signal there is in general at least one restriction site or a polylinker which enables the insertion of a coding DNA sequence. The DNA sequence naturally controlling the transcription of the corresponding gene can be used as the promoter sequence if it is active in the selected host organism. However, this sequence can also be exchanged for other promoter sequences. It is possible to use promoters ensuring constitutive expression of the gene and inducible promoters which permit a deliberate control of the expression of the gene. Bacterial and viral promoter sequences possessing these properties are described in detail in the literature. Regulatory sequences for the expression in microorganisms (for instance *E. coli, S. cerevisiae)* are sufficiently described in the literature. Promoters permitting a particularly high expression of a downstream sequence are for instance the T7 promoter (Studier, Methods in Enzymology 185, 60-89, 1990), lacUV5, trp, trp-lacUV5 (DeBoer, Promoters, Structure and Function, Praeger, 462-481, 1982; DeBoer, Proc. Natl. Acad. Sci. USA 80, 21-25, 1983), Ip1, or rac (Boros, Gene 42, 97-100, 1986). Inducible promoters are preferably used for the expression of downstream sequences. These promoters often lead to higher polypeptide yields than do constitutive promoters. In order to obtain an optimum amount of polypeptide, a two-stage process is often used. First, the host cells are cultured under optimum conditions up to a relatively high cell density. In the second step, transcription is induced depending on the type of promoter used. In this regard, a tac promoter is particularly suitable which can be induced by lactose or IPTG (=isopropyl-β-D-thiogalactopyranoside) (DeBoer, loc. cit.). Termination signals for transcription are also described in the literature.

The present invention further relates to an organelle comprising the nucleic acid molecule as described herein or the vector as described herein. Thus, the invention provides an organelle comprising a nucleic acid molecule encoding a polypeptide of the invention or a vector comprising a nucleic acid molecule encoding a polypeptide of the invention. Thus, the invention provides an organelle producing the polypeptides of the present invention. An organelle is a specialized subunit within a cell, usually a eukaryotic cell, that performs a specific function. Examples of organelles are the mitochondrion, nucleus, Golgi apparatus, endoplasmic reticulum, and plastids.

The invention also relates to a host cell comprising the herein described nucleic acid molecule, the herein described vector, or the herein described organelle. Thus, the present invention provides a host cell comprising a nucleic acid molecule encoding a polypeptide of the invention, a vector comprising a nucleic acid molecule encoding a polypeptide of the invention, or an organelle comprising a nucleic acid molecule encoding a polypeptide of the invention or a vector comprising a nucleic acid molecule encoding a polypeptide of the invention. The skilled person is readily capable of choosing suitable host cells, e.g., for polypeptide expression according to various factors such as the desired polypeptide, the level of expression required, the downstream processing requirements, and the cost of the process. Commonly used host cells for protein expression include bacteria (such as *E. coli*)*,* yeast (such as *Saccharomyces cerevisiae),* insect cells (such as Sf9 and Sf21), and mammalian cells (such as CHO and HEK293). Each host cell has its advantages and limitations. For example, bacterial systems are relatively easy to grow and produce high yields of protein, but they may not be suitable for expressing complex eukaryotic proteins with post-translational modifications. Yeast systems are also easy to grow and have the ability to perform some post-translational modifications, but they may not be suitable for certain types of proteins. Insect cells have the ability to perform some post-translational modifications. They are suitable for expressing large, complex proteins but are more expensive to culture than bacterial and yeast systems. Mammalian cells are often preferred for producing biologically active and properly folded proteins or polypeptides with complex post-translational modifications. Still, they are more difficult and expensive to culture than bacterial and yeast systems. Ultimately, the choice of the host cell for polypeptide expression will depend on the specific requirements of the polypeptide and the downstream application.

Although evident for the skilled person it is pointed out that when an organelle or host cell comprises a herein described nucleic acid it is envisaged that said organelle or host cell expresses said nucleic acid, i.e. said organelle or host cell produces the polypeptide encoded by the nucleic acid. Accordingly, it is envisaged that an organelle or host cell produces the herein described polypeptides of the present invention. Preferably, the present invention relates to an organelle or host cell produces the polypeptide comprising an amino acid sequence according to SEQ ID NO: 4 or an amino acid sequence of at least 80% sequence identity to SEQ ID NO: 4. The present invention further relates to an organelle or host cell that produces the polypeptide comprising an amino acid substitutions relative to SEQ ID NO: 4 selected from (i) K26N, (ii) K26T, (iii) D14N, R17S, K26N, or (iv) D14N, R17S, K26T. Furthermore, the present invention relates to an organelle or host cell that produces the polypeptide comprising an amino acid sequence selected from the group of SEQ ID NOs: 7, 6, 5, and 8, preferably SEQ ID NO: 7. Accordingly, an organelle of the present invention or a host cell of the present invention preferably comprises a nucleic acid encoding for a encoding an amino acid sequence selected from the group of SEQ ID NO: 4 or an amino acid sequence of at least 80% sequence identity to SEQ ID NO: 4, a nucleic acid molecule encoding an amino acid sequence comprising the amino acid substitutions relative to SEQ ID NO: 4 selected from (i) K26N, (ii) K26T, (iii) D14N, R17S, K26N, or (iv) D14N, R17S, K26T, or a nucleic acid molecule encoding an amino acid sequence selected from the group of SEQ ID NOs: 7, 6, 5, and 8, preferably SEQ ID NO: 7. Accordingly, it is envisaged that an organelle of the present invention or host cell of the present invention produces the polypeptides of the present invention.

It is envisaged that the herein described organelles or host cells are used for certain methods and processes. Accordingly, the invention provides uses of the herein described organelles or host cells. It is pointed out that all disclosed uses and methods for herein described organelles or host cells are also disclosed herein for the described nucleic acids molecules and vectors if they can be used in a context where the encoded polypeptides may be produced.

Accordingly, the present invention relates to a method for producing a polypeptide of the present invention comprising the step of culturing the herein described host cell under conditions in which the polypeptide of the present invention is produced. Accordingly, the present invention provides a method for producing a polypeptide of the present invention comprising the step of culturing a host cell comprising the herein described nucleic acid molecule, the herein described vector, or the herein described organelle under conditions in which the polypeptide is produced. The person skilled in the art is aware of means and methods for culturing a host cell as described herein, including culture conditions and medium required for culturing the respective host cell described herein and conditions in which the polypeptide is produced.

The present invention further provides that the method for producing a polypeptide of the present invention comprising a further step of isolating the polypeptide of the present invention from the cultured host cell, organelle, culture, or the culture medium. The person skilled in the art is further aware of means and methods that allow the isolation of a polypeptide of the present invention from a host cell, organelle, culture, or culture medium.

The present invention further relates to a method of producing the polypeptides of the present invention by means of synthetic or cell-free polypeptide synthesis. The person skilled in the art is aware of suitable means and methods to produce polypeptides synthetically or in a cell-free setup. Examples thereof may include, but are not limited to, solid-phase peptide synthesis (SPPS), solution-phase synthesis (SPS), and cell-free protein synthesis (CFPS). A preferred method of producing the polypeptides of the present invention by means of synthetic or cell-free polypeptide synthesis is solid-phase peptide synthesis (SPPS). An example thereof is illustrated in the non-limiting **Example 1-C.**

In general, polypeptides of up to about forty amino acids, more preferably between about twenty and about forty amino acids, most preferably of about thirty amino acids, can be synthesized using any one of the methods known to the person skilled in the art. Accordingly, the polypeptides of the present invention can be synthesized using any one of the methods known to the person skilled in the art. Particular examples thereof are the herein described cell-based and cell-free or synthetic methods of polypeptide synthesis (i.e., production).

The described polypeptides of the present invention, produced by organelles or host cells as described herein and/or isolated from the host cell, organelle, culture, or culture medium as described herein, or produced by synthetic or cell-free polypeptide synthesis as described herein may be used as a medicament and/or for the treatment and/or prevention of a coronavirus invention as described herein above.

It is also envisaged that the herein described nucleic acids, vectors, organelles, or host cells are comprised in compositions that may, e.g., comprise additional reagents for a specific method or use. The present invention also provides a composition comprising the herein described nucleic acids, vectors, organelles, or host cells.

The herein described uses and methods for the herein described nucleic acids, vectors, or host cells, are disclosed for the corresponding compositions or pharmaceutical compositions *mutatis mutandis.*

In another aspect, the present invention relates to a method for inhibiting cell entry of coronavirus into a target cell comprising the step of contacting the target cell with a polypeptide of the present invention. In particular, the present invention provides a method for inhibiting cell entry of coronavirus into a target cell comprising the step of contacting the target cell with a polypeptide of the present invention, wherein the target cell is *in vivo, in vitro,* or *ex vivo.* Methods for evaluating the inhibition of cell entry are known in the art. As laid out herein above, the person skilled in the art is aware that inhibitory effects of an agent, such as a polypeptide of the present invention, can be determined, for example *in vitro* or *ex vivo,* illustrative examples are provided herein in the appended non-limiting examples. Methods for determining inhibitory effect of an agent are known in the art. A non-limiting example for an *in vitro* method for measuring the inhibitory effect of a polypeptide may be as described by Schwarze and colleagues (Schwarze et al. (2022). Frontiers in immunology, 13, S. 915034), such as incubating microplates coated with RBD protein (for example SARS-CoV-2 RBD protein) with a solution containing ACE-2 and the polypeptides to be tested, determining the binding of the ACE-2 and RBD protein, for example by densitometric means, such as DO₄₅₀ measurement. Control well would be incubating microplates coated with RBD protein either without polypeptide (negative control, 0% inhibition) or without ACE-2 (positive control, 100% inhibition). To determine and normalize the residual binding (%) and Inhibition (%) the following exemplary but non-limiting calculation may be used: Residual binding (%) = 100 x ((sample OD₄₅₀ - positive control OD₄₅₀) / (negative control OD₄₅₀ - positive control OD₄₅₀)); Inhibition (%) = 100 - residual binding (%); from mg/L to µmol/L: (X mg/L / X Da (monoisotopic polypeptide mass)) / 1000 = X µmol/L; (see also, e.g., non-limiting **Example 1-E,** or Schwarze et al. (2022)). A non-limiting example for an *ex vivo* or *in vitro* method for measuring the inhibitory effect of a polypeptide may be seeding a fixed number of target cells (e.g., HEK293) of a tissue culture and incubating the culture with a relevant virus (for example a SARS-CoV-2 pseudovirus). After a set period of incubation the infection rate of the tested virus is evaluated (by methods known in the art). Such measuring the inhibitory effect of a polypeptide may be conducted with commercially available test kit, a non-limiting example of such a kit would be the "COVID-19 Pseudovirus Neutralizing Antibody Assay (Luciferase)" from Abnova Corporation (Catalog Number KA6152); see also, e.g., non-limiting **Example 1-H.** As laid out herein, in context of the present invention inhibition (%) of the polypeptides of the present invention may be preferably determined by the herein described pseudovirus assay and will range between about 30 % and 100 %, preferably at least about 30%, at least 40%, at least about 50%, at least about 60 %, at least about 70%, at least about 80%, at least about 85%, at least about 90% at least about 95%, at least about at least about 96%, at least about 97%, at least about 98%, at least about 99%, or 100%. However, the person skilled in the art is aware that the effective inhibition of a polypeptide may optionally be determined by other means and methods, some of which are also further described herein, such as the RBD-ACE2 assay (see, e.g., **Example 1-E).** It is envisaged that the effectiveness of the polypeptide is preferably determined by the determination of the IC₅₀ concentration, preferably by the methods described herein.

In addition, the present invention provides means (e.g., kits) which may be used in the methods according to the present invention.

Thus, in another aspect, the present invention provides a kit comprising a polypeptide of the present invention, a composition comprising a polypeptide of the present invention, a pharmaceutical composition comprising a polypeptide of the present invention, a vector comprising a nucleic acid molecule comprising a gene encoding a polypeptide of the present invention, or a host cell comprising a nucleic acid molecule comprising a gene encoding a polypeptide of the present invention or the vector comprising a nucleic acid molecule comprising a gene encoding a polypeptide of the present invention.

Additionally, the present invention provides a kit-of-parts comprising (i) a polypeptide of the present invention, a composition comprising a polypeptide of the present invention, a pharmaceutical composition comprising a polypeptide of the present invention, a vector comprising a nucleic acid molecule comprising a gene encoding a polypeptide of the present invention, or a host cell comprising a nucleic acid molecule comprising a gene encoding a polypeptide of the present invention or the vector comprising a nucleic acid molecule comprising a gene encoding a polypeptide of the present invention and (ii) one or more further antiviral agent(s). In context of the present invention, the further antiviral agent is not particularly limited, and examples thereof may be mRNA vaccines, adenovirus vector vaccines, inactivated virus vaccines, subunit vaccines, monoclonal antibody therapies or other small molecule treatments. Preferably, the one or more further antiviral agent(s) is selected from the group of Remdesivir, Nirmatrelvir, Ritonavir, Dexamethasone, and Tocilizumab.

In context of the present invention, the kit or kit-of-parts may further comprise a brochure or leaflet with instructions for its use, for example, in a method according to the present invention.

The disclosures in context of the methods described herein are disclosed as corresponding uses *mutatis mutandis.* The disclosures in context of the uses described herein are disclosed as corresponding methods *mutatis mutandis.*

The term "method" refers to manners, means, techniques and procedures for accomplishing a given task including, but not limited to, those manners, means, techniques and procedures either known to, or readily developed from known manners, means, techniques and procedures by practitioners of the chemical, biological and biophysical arts.

Unless otherwise defined, all terms of art, notations and other scientific terminology used herein are intended to have the meanings commonly understood by those of skill in the art to which the present invention pertains. In some cases, terms with commonly understood meanings are defined herein for clarity and/or for ready reference, and the inclusion of such definitions herein should not necessarily be construed to represent a difference over what is generally understood in the art. The techniques and procedures described or referenced herein are generally well understood and commonly employed using conventional methodologies by those skilled in the art. As appropriate, procedures involving the use of commercially available kits and reagents are generally carried out in accordance with manufacturer-defined protocols and conditions unless otherwise noted.

As used herein, the singular forms "a," "an," and "the" include the plural referents unless the context clearly indicates otherwise. Accordingly, the articles "a", "an", and "the" refer to one or to more than one (i.e., to at least one) of the grammatical object of the article. By way of example, "an element" means one element or more than one element. The terms "include", "such as" and "and the like" are intended to convey inclusion without limitation, unless otherwise specifically indicated.

As used herein, the term "or" is generally employed in its usual sense including "and/or" unless the content clearly dictates otherwise. The term "and/or" refers to and encompasses any and all possible combinations of one or more of the associated listed items, as well as the lack of combinations when interpreted in the alternative (or).

As used herein, the term "about" indicates and encompasses an indicated value and a range above and below that value. For example, the term "about" may indicate the designated value ±10%, ±9%, ±8%, ±7%, ±6%, ±5%, ±4%, ±3%, ±2%, or ±1% without substantial loss of activity or effect. In certain embodiments, where applicable, the term "about" indicates the designated value(s) ± one standard deviation of that value(s).

As used herein, the term "at least" means the specified value, quantity, or characteristic is the minimum required, but it does not exclude the presence of greater values, additional elements, or further enhancements beyond what is expressly stated. In particular, "at least," when referring to a numerical value, indicates a minimum threshold that must be met or exceeded. The specified value represents the lower boundary, and greater values are encompassed within the scope of the disclosure unless explicitly stated otherwise. Accordingly, "at least," when used in reference to the quantity of a component, signifies that the stated minimum number is required, but additional instances of the component may be included without departing from the scope of the invention. Also, "at least," in relation to a performance parameter, denotes that the specified value is the minimum level achieved or maintained, while higher levels of performance are within the scope of the invention. Additionally, "at least," when describing structural features or procedural steps, means that the specified minimum number of elements or steps is required, but additional elements or steps may be included.

The above is furthermore illustrated in the appended non-limiting examples.

### Brief Description of Drawings

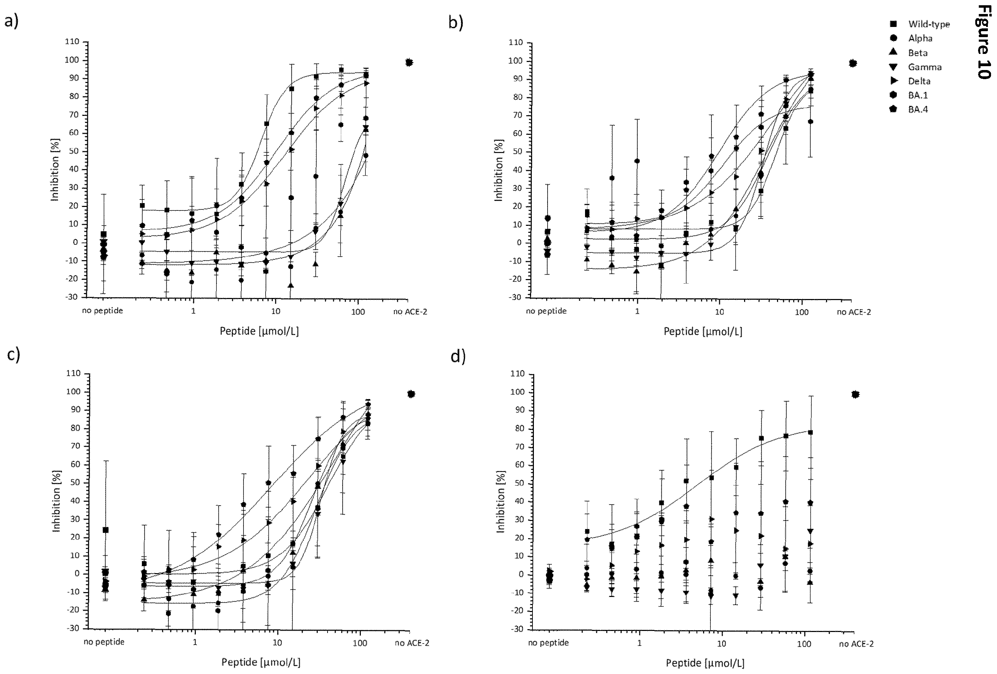

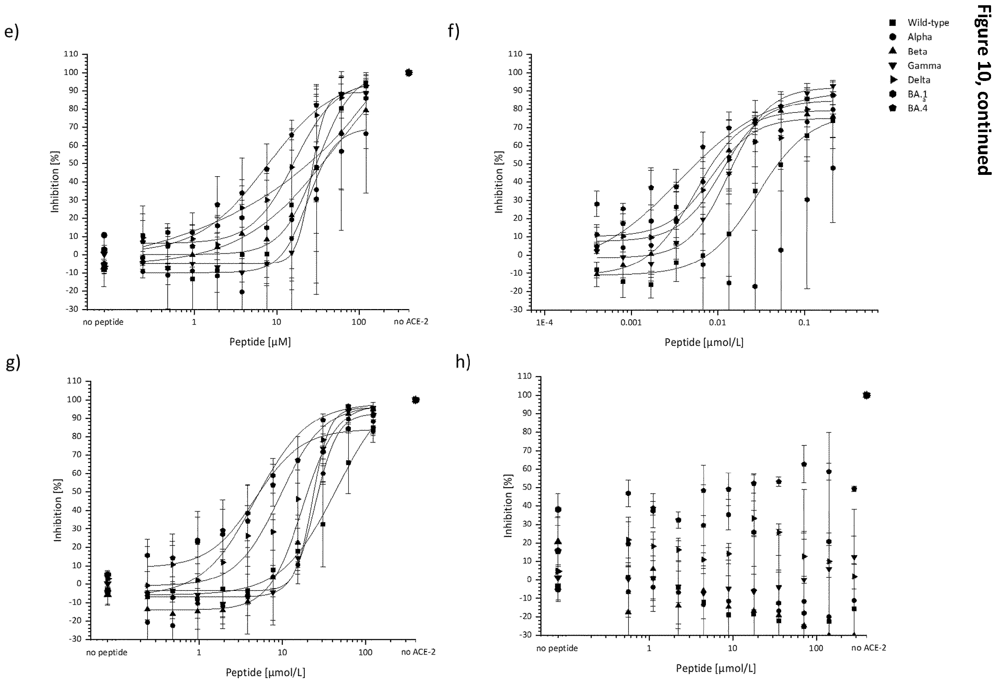

The invention is further described by reference to the following illustrative and non-limiting figures. The appended figures show:
**Figure 1** - **Sequences, monoisotopic masses, purities, K_{d}- and ICso-values of all synthetic polypeptides.**
   The abbreviations n/i, n/a, and n/b denote not calculable IC₅₀, data not available, and no binding observed, respectively.
**Figure 2** - **K_{d}-values for polypeptide binding to ACE-2 and ICso-values for polypeptide inhibition of ACE-2 binding to wild-type RBD.**
   The top graphs **(a)** illustrate the K_{d}- (circles) and IC₅₀-values (squares) for the polypeptides tested, with the exact data shown in the tables below **(b).** The polypeptide codes are shown on the outside of the graph. The gray dotted lines indicate the separation of the different sequences. In the tables, polypeptides with no detectable effect are indicated as n/b (no binding) or n/i (no calculable inhibition / IC₅₀) and polypeptides positive in the control experiment are indicated as n/a (not accessible).
**Figure 3** - **RBD-ACE-2 inhibition assay of wild type polypeptides.**
   Shown are the absorbance at 450 nm **(a)** and the normalized inhibition **(b)** of wild-type RBD, four polypeptides derived from wild-type RBD, and two control polypeptides, one originated from the N-protein and the antimicrobial polypeptide (AMP) LL-37 competing with recombinant wild-type RBD (coated) for binding to the ACE-2 receptor (preincubated with polypeptide or protein) measured as triplicates.

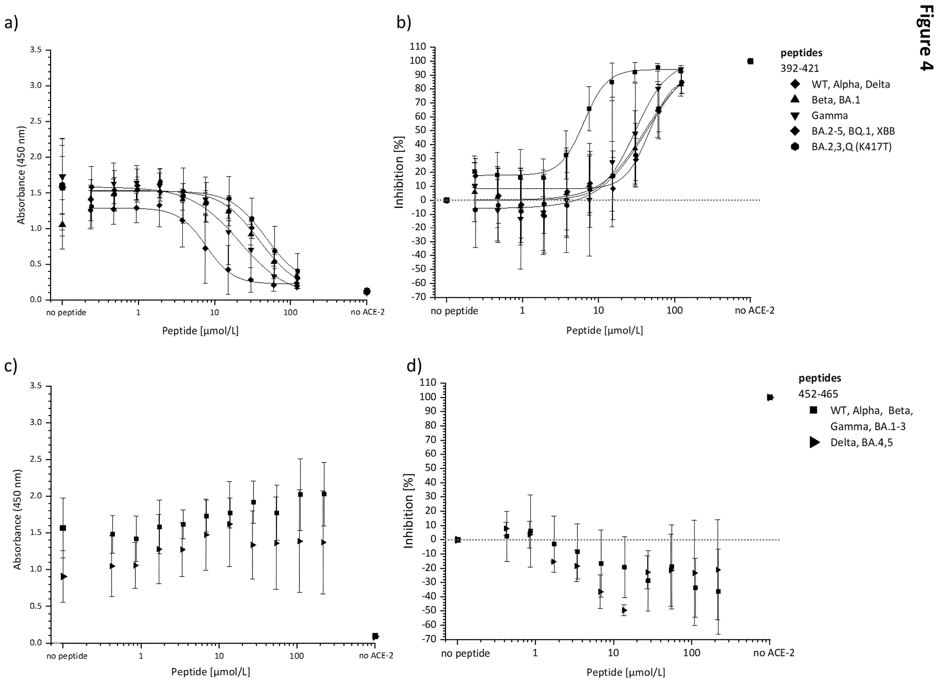

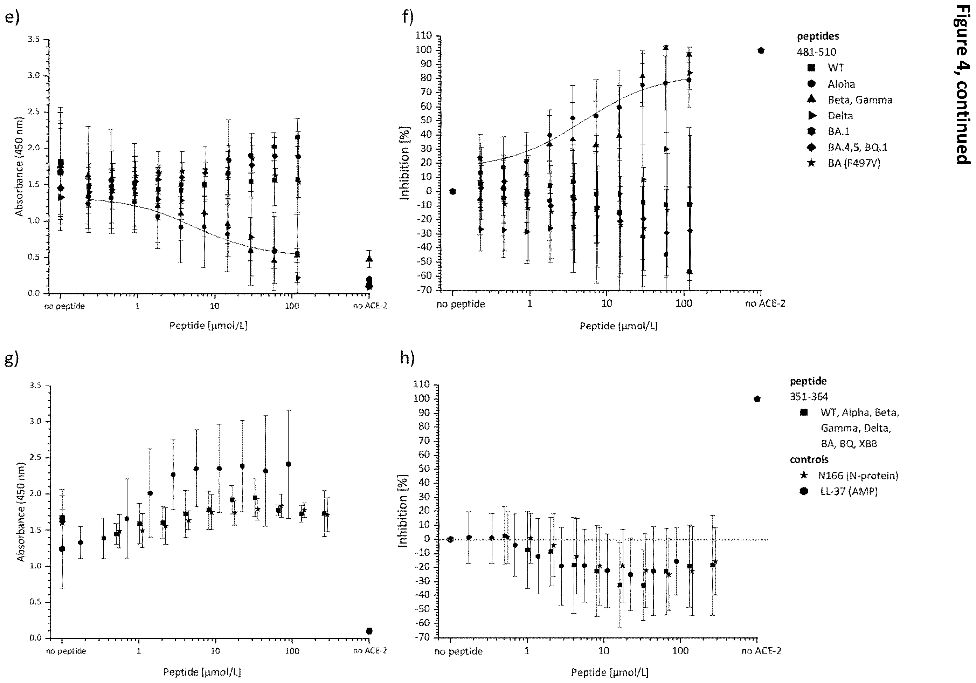

**Figure 4** - **RBD-ACE-2 inhibition assay of VOC polypeptides.**
   Shown are the absorbances at 450 nm **(a, c, e, g)** and the normalized inhibition **(b, d, f, h)** of 15 polypeptides derived from RBD sequences 392 to 421 **(a, b),** 452 to 465 **(d, e),** 481 to 510 **(d, e),** and 351 to 364 **(g, h)** of wild-type, alpha, beta, gamma, delta, and omicron variants BA.1 to BA.4 VOCs, and control polypeptides N166 and LL-37 **(g, h)** competing with recombinant wild-type RBD (coated) for binding to the ACE-2 receptor (preincubated with polypeptide) measured as triplicates.

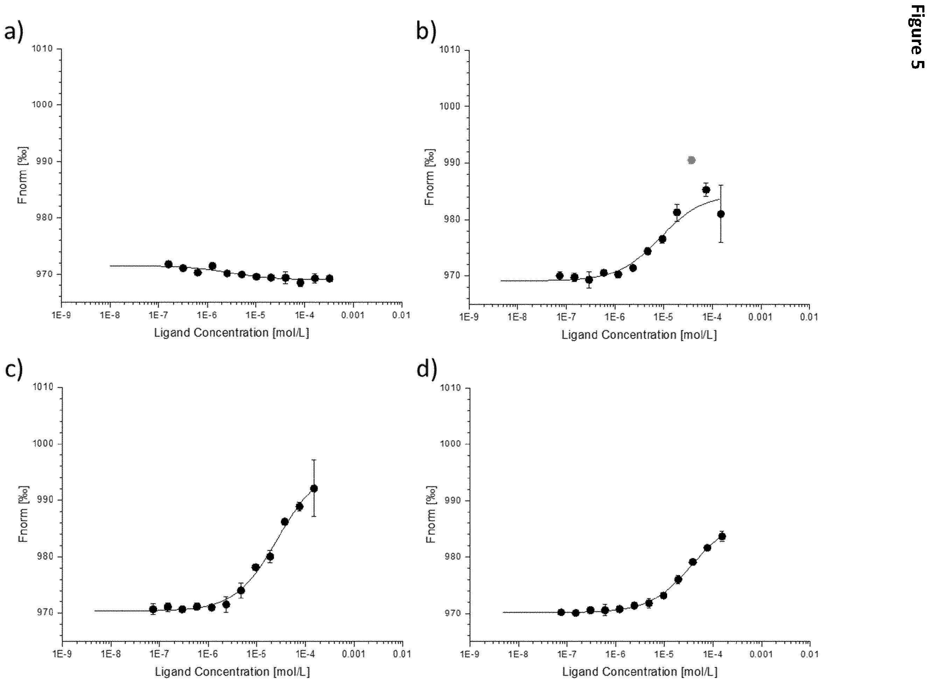

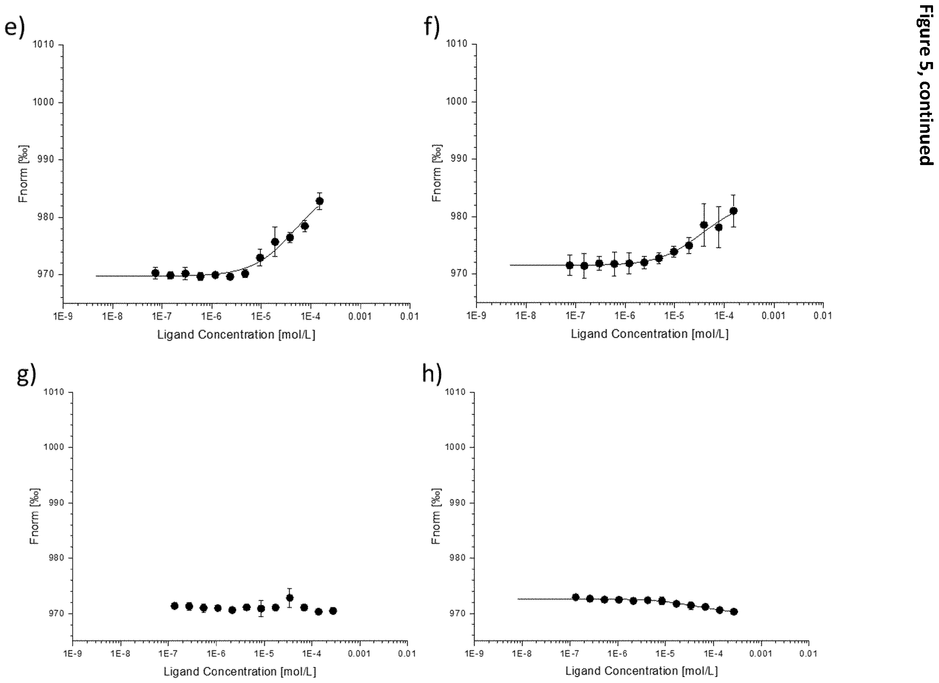

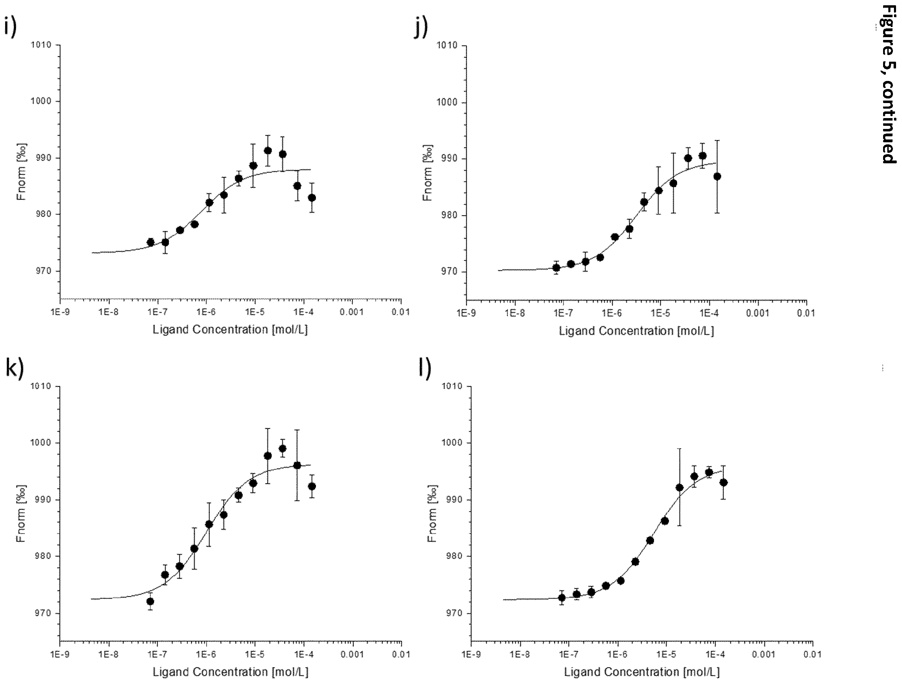

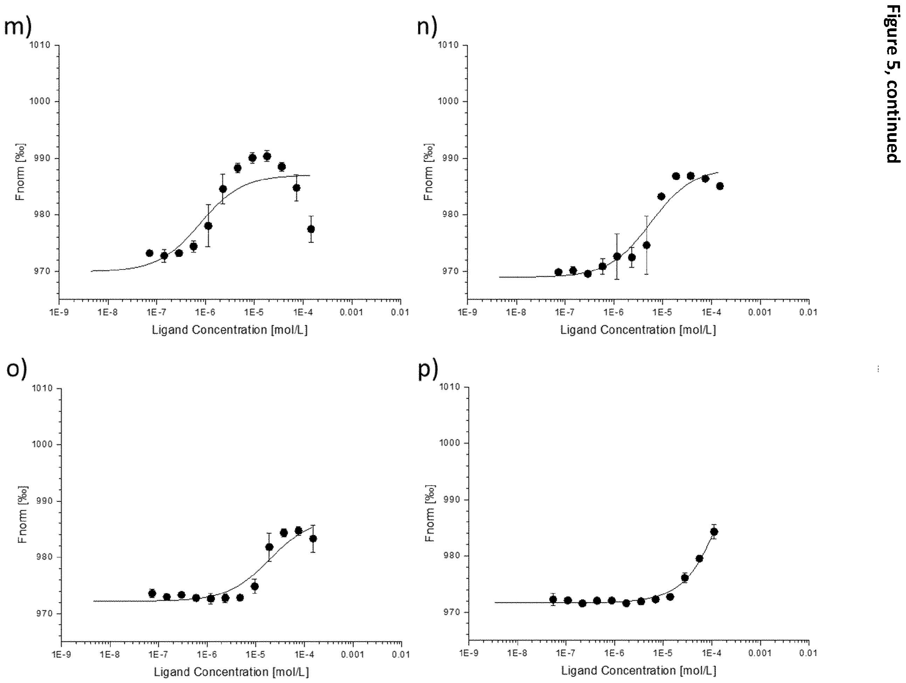

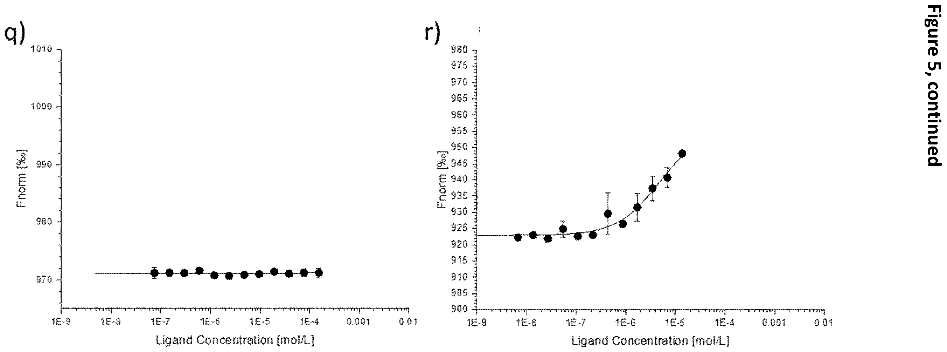

**Figure 5** - **Microscale thermophoresis fitted dose response curves for polypeptides.** p351wt **(a),** p392wt **(b),** p392b **(c),** p392g **(d),** p392BA.2 **(e),** p392BA.2T **(f),** p452wt **(g),** p452d **(h),** p481wt **(i),** p481a **(j),** p481b **(k),** p481d **(l),** p481BA.1 **(m),** p481BA.4 **(n),** and p481F497V **(o)** and controls consisting of polypeptide LL-37 **(p),** polypeptide pNP66 **(q),** and recombinant wild-type RBD **(r)** binding to the recombinant ACE-2 receptor (37°C, 100% LED/excitation power, low MST power) using the software MO.Control (version 1.5.1). Fitting was performed using MO.Affinity Analysis (version 2.2.5) and data points corresponding to outliers (highlighted in gray) were not used in the calculation. The normalized fluorescence MST traces were acquired with an on-time of 1.5 s for polypeptides and 20 s for wild-type RBD.

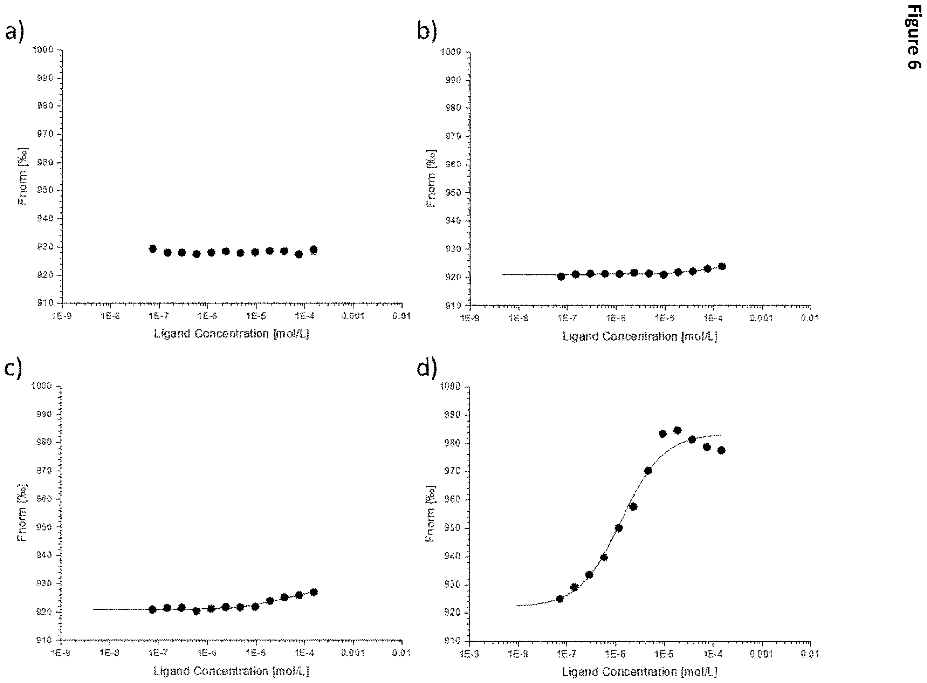

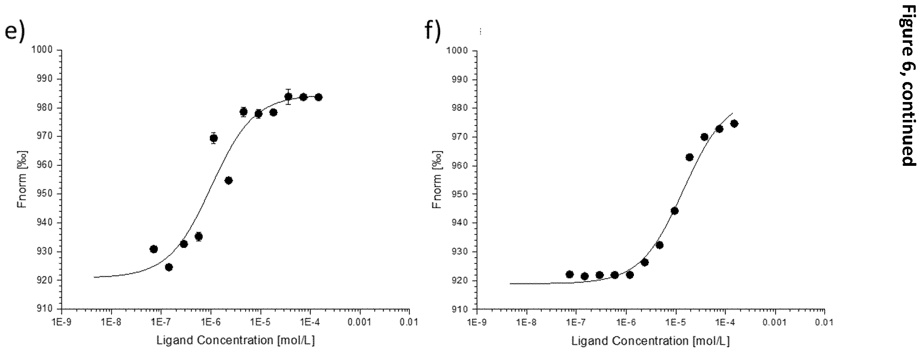

**Figure 6** - **Microscale thermophoresis fitted dose response curves for diluted polypeptides.**
   p392wt **(a),** p392g **(b),** p392BA.2T **(c),** p481wt **(d),** p481BA.1 **(e),** and p481F497V **(f)** mixed with labeled His6 control polypeptide at 37°C (100% LED/excitation power, low MST power) using the software MO.Affinity Analysis (version 2.2.5). The normalized fluorescence MST traces were acquired with an on-time of 1.5 s. The polypeptides were analyzed in the presence of the control polypeptide due to strong fluctuation and increase of fluorescence during initial capillary scans.
**Figure 7** - **Normalized binding of ACE-2 receptor to coated RBDs of wild-type SARS-CoV-2 and** six **common VOCs.**
   Shown is a twofold serial dilution series of all polypeptides from 400 mg/L down to 0.78 mg/L and full-length RBD from 100 mg/L to 0.2 mg/L. Each row corresponds to a polypeptide and each column corresponds to a mutant RBD coated in the inhibition assay. The IC₅₀ values are indicated by vertical dotted lines and are shown in each graph with the standard deviation, if applicable (n/i no calculable inhibition/IC₅₀). The original data are shown in **Figures 8 to 9****.**
**Figure 8** - **RBD-ACE-2 inhibition assay using VOC RBDs and polypeptides p392 and p481a.**
   Shown are the absorbances at 450 nm **(a, c, e)** and normalized inhibition **(b, d, f)** of the full length wild-type RBD **(a, b, e, f)** and beta RBD **(c, d**) , six polypeptides derived from RBD sequences 392 to 421 of wild-type (squares, identical to the RBD of alpha and delta VOCs), beta (up triangle, identical to the RBD of omicron BA.1), gamma (down triangle), omicron BA.2-5 (hexagon, identical to omicron variants BQ.1 and XBB), and omicron BA.2 (diamond, identical to omicron variants BA.2, BA.3 and BA.Q with K417T) SARS-CoV-2 variants, one polypeptide derived from RBD sequences 481 to 510 of the alpha SARS-CoV-2 variant (p481a, circles) and the control polypeptide N66 (star) competing with recombinant wild-type **(a, b),** beta **(c, d),** and omicron BA.4 **(e, f)** RBD variants (coated) for binding to the ACE-2 receptor (preincubated with polypeptide or protein). Since RBD variant BA.1 was not available in sufficient quantities, the assay was incubated with wild-type RBD, which did not inhibit binding.

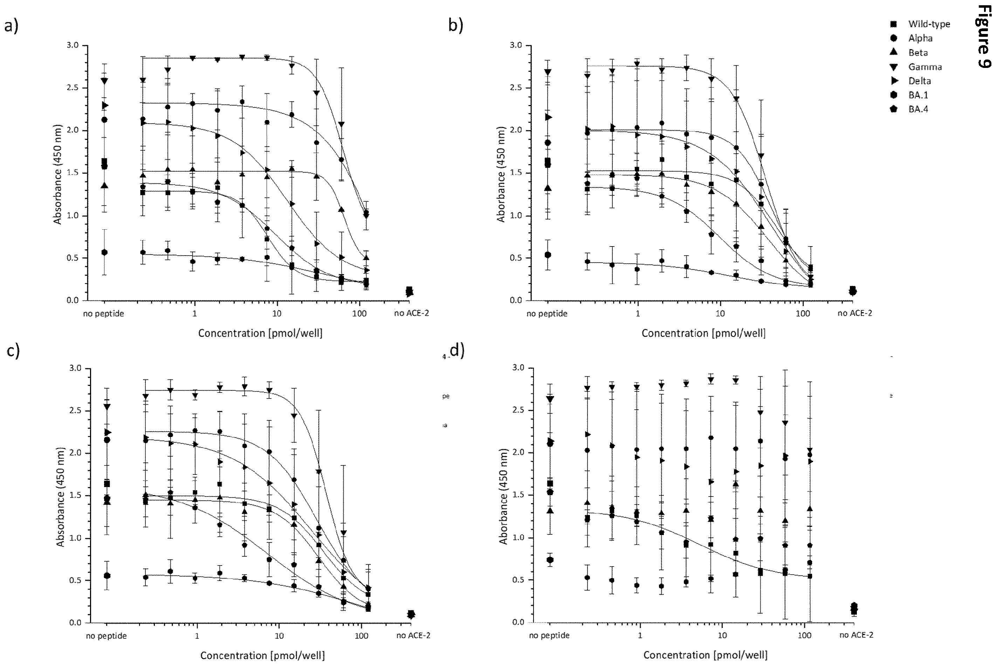

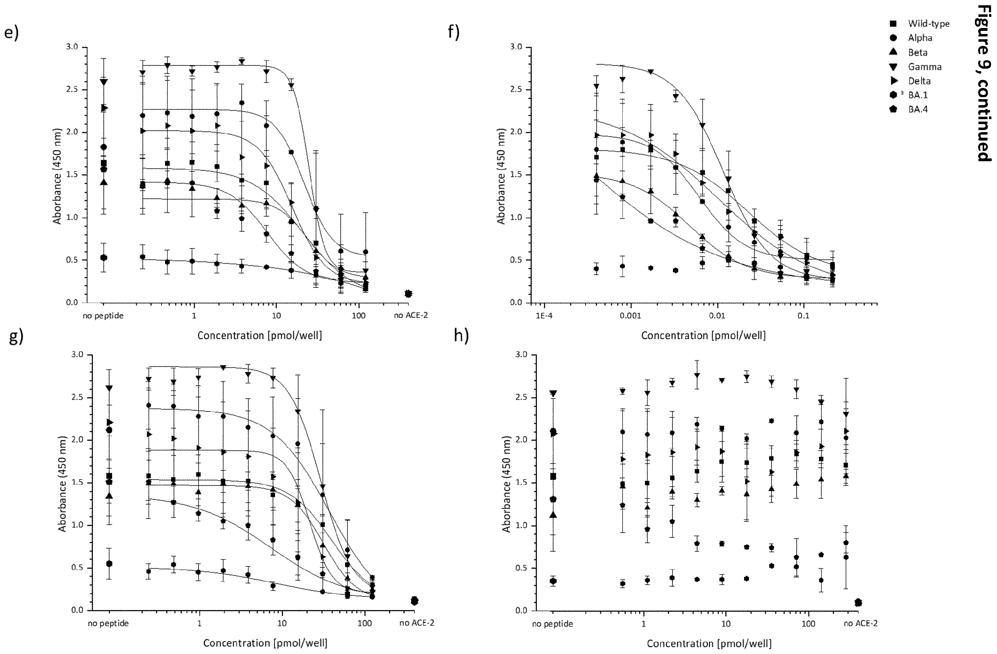

**Figure 9** - **RBD-ACE-2 inhibition assay.**
   Shown are the absorbances at 450 nm for polypeptides p392wt **(a),** p392b **(c),** p392g **(e),** p392BA.2 **(g),** p392BA.2T **(b),** and p481a **(d),** RBD proteins **(f),** and polypeptide pNP66 **(h)** competing with recombinant RBD variants (coated) for binding to the ACE-2 receptor (preincubated with polypeptide or protein). The legend shows all coated RBD proteins with their corresponding symbols. The normalized inhibitions are shown in **Figure 10****.**
**Figure 10** - **RBD-ACE-2 inhibition assay.**
   Shown are the normalized inhibitions for polypeptides p392wt **(a),** p392b **(c),** p392g **(e),** p392BA.2 **(g),** p392BA.2T **(b),** and p381a **(d),** RBD proteins **(f),** and polypeptide pNP66 **(h)** competing with recombinant RBD variants (coated) for binding to the ACE-2 receptor (preincubated with polypeptide or protein). The legend shows all coated RBD proteins with their corresponding symbol. The recorded absorbances are shown in **Figure 9****.**
**Figure 11** - **Pseudovirus assay of five relevant p392 polypeptides, p481a, control polypeptide pNP66, recombinant wild-type RBD, and an anti-SARS-CoV-2 monoclonal antibody.**
   The controls consisted of polypeptides p481a and pNP66, the wildtype RBD protein, and the SARS-CoV-2 mAb. The relative luminescence units (RLU) correspond to the infection rate of the pseudovirus for different polypeptide concentrations in mg/L. The dashed lines indicate a broken coordinate axis. Measurements were performed in two biological replicates.
**Figure 12** - **Bar graph showing OD₄₅₀ values of the RBD IgG ELISA.**
   OD₄₅₀ values of the RBD IgG ELISA were obtained by adding a polypeptide **(a, b)** or the recombinant wild-type RBD **(b)** to serum to compete with antibody recognition of the coated wild-type RBD as positive control. The negative control corresponds to no added serum.
**Figure 13** - **Viable cell counts obtained in the cytotoxicity assay.**
   Shown are viable cell counts for ACE-2 overexpressing HEK293 cells using the CASY system for all polypeptides (800 mg/L) and wild-type RBD (200 mg/L) used in the pseudovirus assay **(a)** and the most potent polypeptide p392wt in the absence or presence of pseudovirus **(b).** The positive control was (12% (v/v) DMSO) and the negative controls were 12% (v/v) PBS and DMEM medium. Viable cell counts were determined in solution after cells were detached from the well. The dashed lines indicate a broken coordinate axis and the dashed-dotted line indicates the value of the negative control (DMEM medium).
**Figure 14** **- Serum stability assay.**
   Serum stability assay corresponding to the RBD regions 392 to 421, 452 to 465, and 481 to 510. Shown are the polypeptide quantities obtained after incubation periods of 0 (left, dotted bars), 45 (middle, dashed bars), and 90 (right, black bars) minutes in human serum relative to the initial polypeptide quantity. Serum was diluted 100-fold in PBS buffer. Assays were performed in triplicate.

### Examples

To provide a better understanding of the present invention and its many advantages certain embodiments of the invention will now be described with reference to the following examples, which are intended for the purpose of illustration only and are not intended to limit the scope of the generality hereinbefore described.

### Example 1- General Experimental Methods

Reagents were obtained from the following manufacturers:
- Abnova Corporation (Taipeh, Taiwan): COVID-19 Pseudovirus Neutralizing Antibody Assay (Luciferase, Cat# KA6152), SARS-CoV-2 Monoclonal Antibody (Cat# MAB22854-M07, clone 4E10);
- Antikörper-online.de (Aachen, Germany): SARS-CoV-2 Spike Protein (BA.4 - Omicron, BA.5 - Omicron, RBD, His Tag);
- Biosolve BV (Valkenswaard, Netherlands): Dimethylformamide (DMF, polypeptide synthesis grade) and piperidine (≥ 99.5%);
- Carl Roth GmbH & Co. KG (Karlsruhe, Germany): Carbenicillin disodium salt, ROTI^{®}Stock 10x PBS, ROTI^{®}Stock 10x PBS-T, sodium chloride (≥ 99.5%), sodium dodecyl sulfate (SDS, ≥ 99.5%), and sulfuric acid;
- GenScript (Leiden, Netherlands): SARS-CoV-2 Spike protein RBD (Omicron BA.1 Variant, His Tag); Honeywell FlukaTM (Seelze, Germany): Ethane-1,2-dithiol;
- NanoTemper Technologies GmbH (München, Germany): Monolith His-Tag Labeling Kit RED-tris-NTA 2nd Generation (Cat# MO-L018);
- Promega GmbH (Mannheim, Germany): Luciferase Assay System (Cat# E1500), and Peroxidase-conjugated anti-human IgG antibody;
- Seramun Diagnostika GmbH (Heidesee, Germany): TMB substrate solution;
- SERVA Electrophoresis GmbH (Heidelberg, Germany): Acrylamide/bis(acrylamide) (30% T, 2.67% C), ammonium persulfate (APS; analytical grade), BlueBlock PF 10x, Coomassie Brilliant Blue G-250, and N,N,N',N'-tetramethylethane-1,2-diamine (TEMED, analytical grade);
- Sigma Aldrich Chemie GmbH (Taufkirchen, Germany): 2-Mercaptoethanol (BioUltra), 1-hydroxybenzotriazole (HOBt, ≥ 97%), m-cresol (≥ 99%), Cell Dissociation Solution Non-enzymatic 1x, N,N'-diisopropylcarbodiimide (DIC, ≥ 98%), ExtrAvidin^{®}-Peroxidase, imidazole (≥ 99.5%), polyethylenimin (PEI), and trifluoroacetic acid (TFA, for HPLC, > 99%);
- Sino Biological (Eschborn, Germany): Angiotensin-Converting Enzyme 2 Protein (His Tag, Biotinylated;
- Surmodics IVD, Inc. (Eden Prairie, USA): Assay diluent (Protein-Free) and StabilZymeTM SELECT;
- Thermo Fisher Scientific (Waltham, Massachusetts, USA): SuperBlock^{®} (PBS), Gibco DMEM, Gibco Fetal Bovine Serum (FBS), Gibco GlutaMAX Supplement, and Gibco 100x MEM non-essential amino acids solution;
- VWR International GmbH (Darmstadt, Germany): Acetonitrile (HPLC-gradient grade), diethyl ether, and formic acid (98%).

### A) Serum collection

Serum samples (n=7) were collected in December 2021 from seven uninfected individuals vaccinated three times with mRNA-based vaccines BNT162b2 and/or mRNA-1273.

### B) Recombinant proteins expressed in HEK cells

The target sequence obtained from a pUC vector (RBD 318-541bp, GenScript) was cloned into the pHLsec expression vector (Aricescu et al. (2006). Acta crystallographica, Pt 10, S. 1243-1250) with a C-terminal His-tag. The SARS-CoV-2 variant vectors were generated by single amino acid mutagenesis (QuikChange mutagenesis) in the pUC vector to insert the mutation sites for alpha RBD (B.1.1.7: N501Y), beta RBD (B.1.351: K417N, E484K, N501Y), delta RBD (B.1.617.1, later reclassified as kappa RBD: E484Q, L452R), and gamma RBD (B.1.1.28 P.1: K417T, E484K, N501Y). Variants B.1.1.529.1 (Omicron BA.1) and B.1.1.529.4/5 (Omicron BA.4/5) RBD were purchased as purified C-terminally His-tagged proteins (GenScript, Antibodies-online GmbH). For transient transfection of RBDs into HEK293S cells, plasmid vector (3 mg) and PEI (4.5 mg) were incubated in DMEM (150 mL; 10 mmol/L MEM Non-Essential Amino Acids Solution, 2 mmol/L GlutaMAX) at room temperature (RT). After 15 min, transfection mix (25 mL) and penicillin/streptomycin (10,000 U/mL) solution (2.5 mL) diluted in DMEM medium consisting of 225 mL DMEM containing fetal bovine serum (FBS, 10%) were added to the HEK293S cells in each RollerBottle (Greiner Bio-One GmbH, Frickenhausen, Germany) and incubated at 37°C for 5 days. The medium was harvested and the protein purified by immobilized metal ion affinity chromatography using a HisTrapTM HP column (GE Healthcare, Chicago, IL, USA) according to the manufacturer's guidelines. The RBDs were further purified by size exclusion chromatography (SEC), as recently reported (Schwarze et al. (2022). Frontiers in immunology, 13, S. 915034).

### C) Solid phase polypeptide synthesis

Polypeptides were synthesized by Fmoc/tBu-chemistry on Rink amide resin as C-terminal amides using a SYRO 2000 multiple synthesizer (MultiSynTech GmbH, Witten, Germany). Protected amino acids were activated in situ with DIC/HOBt. Polypeptides were cleaved with TFA containing 12.5% (v/v) of scavenger mixture (ethane-1,2-dithiole, m-cresol, thioanisol, and water, 1:2:2:2, (v/v)), precipitated with ice-cold diethyl ether, and dried. Polypeptides were purified by RP-HPLC using a linear acetonitrile gradient in the presence of 0.1% TFA. Polypeptide masses were confirmed by ESI-MS and the purities were determined by RP-HPLC recording the absorbance at 214 nm.

### D) Enzyme-linked immunosorbent assay (ELISA)

Medium-binding microplates (Greiner Bio-One; 12xF8, PS, F-bottom) were coated with SARS-CoV-2 RBD protein (75 ng/well) in PBS containing NaCl (0.1 mol/L) at 4°C overnight. Wells were washed three times with PBS-T (300 µL/well) and blocked with Superblock (200 µL/well) at RT for 1 h. Equal volumes of serum samples from seven patients vaccinated three times with a mRNA vaccine were pooled and diluted 500-fold in Assay diluent. Polypeptides (400-0.78 mg/L) or RBDs (100-0.20 mg/L) were dissolved in the diluted serum pool, added to the plate (100 µL/well), and incubated at RT for 45 min. Wells were washed three times with PBS-T (300 µL/well), anti-human IgG-HRP (100 µL/well; diluted 25,000-fold in Stabilzyme) was added, and incubated at RT. After 30 min, the solution was removed, the wells were washed three times with PBS-T (300 µL/well), and TMB substrate solution was added (100 µL/well). After 10 min, the reaction was stopped by adding sulfuric acid (0.5 mol/L; 100 µL/well). Absorbance at 450 nm (OD₄₅₀) was recorded using a microplate reader (Molecular Devices, Munich, Germany; SpectraMax Paradigm).

### E) ACE-2 inhibition assay

Medium-binding microplates (Greiner Bio-One; 12xF8, PS, F-bottom) were coated with SARS-CoV-2 RBD protein (37.5 ng/well) in PBS containing NaCl (0.1 mol/L) at 4°C overnight. Blocking was performed as described above for the ELISA. In parallel, polypeptide or protein solutions were prepared as described above in PBS and incubated with ACE-2 (100 ng, 100 µL/well) in PBS (37°C, 45 min). The blocking solution was discarded, the wells were washed three times with PBS-T (300 µL/well), the preincubated ACE-2 polypeptide solution (100 µL) was added, and the plate was incubated at 37°C for 45 min. The wells were washed three times with PBS-T (300 µL/well). ExtrAvidin-Peroxidase (100 µL/well biotin-avidin, diluted 2,000-fold in Stabilzyme) was added and the plate was incubated for 30 min at RT. Subsequent washing steps and color development were identical to the ELISA protocol described above. Control wells were treated similarly to the serum samples, but without addition of polypeptide (negative control, 0% inhibition) or ACE-2 receptor (positive control, 100% inhibition). The mean OD₄₅₀ without ACE-2 from each plate was used as the positive control.

Data were normalized to both controls:
Residual binding (%) = 100 x ((sample OD₄₅₀ - positive control OD₄₅₀) / (negative control OD₄₅₀ - positive control OD₄₅₀)); Inhibition (%) = 100 - residual binding (%); from mg/L to µmol/L: (X mg/L / X Da (monoisotopic polypeptide mass)) / 1000 = X µmol/L.

### F) Microscale thermophoresis (MST)

ACE-2 protein (0.1 µmol/L) was labeled via its His-tag with RED-tris-NTA 2nd Generation dye (0.1 µmol/L), incubated at RT for 30 min, and centrifuged (16,000 × g) according to the manufacturer's instructions. Polypeptides were diluted up to 12-fold with a starting concentration of 1 g/L. Labeled ACE-2 protein (40 nmol/L) was added to equal volumes of the polypeptide dilutions. The final assay concentrations were 20 nmol/L for ACE-2 and 0.5 g/L (327 to 145 µmol/L, depending on the molecular mass of the polypeptide) to 24 µg/L (0.16 to 0.07 µmol/L) for the polypeptide. Samples were transferred to Monolith NT.115 Standard Treated capillaries (K002) and measured on the Monolith NT.115 at 37°C (100% LED/excitation power, low MST power). Data were recorded and processed using the MO.Control 1.5.1 and MO.Affinity Analysis 2.2.5 (NanoTemper) software packages. For polypeptides with nonuniform fluorescence, extended measurements were performed using the His6 control polypeptide included in the kit. The labeled control polypeptide (0.1 µmol/L) was measured using the same protocol, but with 40% LED/excitation power and medium MST power as described in the manual (NanoTemper Technologies. Inc. (2019)).

### G) Serum stability test

Polypeptides (0.6 g/L in PBS; 60 µL) were mixed with diluted serum pool (1:100 in PBS; 100 µL) and incubated in triplicate at 37°C for 0, 45, or 90 min. For protein precipitation, 90% aqueous acetonitrile containing 0.1% formic acid (120 µL was added to the aliquots (30 µL). The samples were incubated on ice for 30 minutes, and centrifuged (12,100 × g) for 5 min. The supernatant (120 µL) was transferred to a new tube, dried under vacuum, and dissolved in methanol (20 µL). After a short incubation period, the sample was diluted with 3% aqueous acetonitrile containing 0.1% formic acid (130 µL). The negative control (diluted serum pool without polypeptide) was treated similarly in parallel. Samples were diluted three times in 3% aqueous acetonitrile containing 0.1% formic acid prior to analysis by RP-HPLC using a Jupiter C18-column (length: 15 cm, internal diameter 2.0 mm, particle size 5 µm, pore size 300 Å, Phenomenex Inc., Aschaffenburg, Germany) and a linear acetonitrile gradient (from 3 to 60 % acetonitrile in 30 min) (Waters GmbH, Eschborn, Germany; ACQUITY Arc System with 2489 UV/VIS detector). Absorbance was recorded at 214 nm and resulting peak areas used for polypeptide quantitation. Polypeptide masses were confirmed online by ESI-Ion Trap-MS (AB Sciex LLC, Framingham, USA; Esquire HCT).

### H) Pseudovirus assay

The pseudovirus assay was performed, as described in the commercial kit instructions (Abnova Corporation (2023)). Briefly, 100,000 HEK293T-hACE-2 cells per well were seeded in a 24-well plate (TPP Techno Plastic Products AG (Trasadingen, Switzerland), TPP^{®} tissue culture plates, flat bottom, polystyrene, 1.86 cm2, sterile) and allowed to grow at 37°C for 195 min. The polypeptide was added to the cells to achieve final concentrations of 0.8 g/L to 0 mg/L and incubated (45 min, 37°C, 5% CO2). In parallel, SARS-CoV-2 pseudovirus was diluted 2.5-fold in DMEM supplemented with 10% FBS, MEM Non-Essential Amino Acids Solution (10 mmol/L) and GlutaMAX (2 mmol/L) and incubated for 30 min at RT. Virus solution (70 µL) was transferred to each well of the plate and incubated (37°C, 5% CO2). After 48 hours, the medium was discarded and the wells were washed with PBS (200 µL/well). Cells were lysed with luciferase cell culture lysis reagent buffer (150 µL/well) and transferred to 1.5-mL tubes on ice. Luciferase Assay Reagent (50 µL) was mixed with lysate (10 µL) and incubated for 1 min. The solution was transferred to a 96-well plate (Greiner Bio-One; PS, F-bottom, white, non-binding) and luciferase expression was measured for 500 ms using a luminescence microplate reader (Molecular Devices, Munich, Germany; SpectraMax Paradigm). The assay was repeated once as a biological replicate on another day.

### I) Cytotoxicity Assay

HEK293T-hACE-2 cells were seeded in a 24-well plate (105 cells/well) and incubated under pseudovirus assay conditions (37°C, 195 min). Polypeptides were added at the highest polypeptide concentration (0.8 g/L) without addition of pseudovirus and incubated for 45 min (37°C, 5% CO2). Wild-type p392-421 was also tested at the highest infection concentration (221 mg/L) in the presence or absence of pseudovirus. For virus supplementation, virus solution (70 µL/well) was added to each well and the plate was incubated for 48 h (37°C, 5% CO2). DMEM medium was used as a control. The cells were detached using non-enzymatic cell dissociation solution (150 µL/well) and counted in each well (viable cells/mL) using the CASY system (Roche Innovatis AG, Bielefeld, Germany; CASY Model TTC - Cell counter and Analyzer, TTC-2JA-2022) (cytena GmbH).

### Example 2 - Design of synthetic RBD-derived polypeptides

Although initial studies on inhibitory polypeptides (Rajpoot et al. (2021). Drugs in R&D, 3, S. 273-283; Rathod et al. (2020). In silico pharmacology, 1, S. 3; Chitsike et al. (2021). Advances in pharmacological and pharmaceutical sciences, S. 1828792; Mahindra et al. (2021). PloS one, 11, e0260283; Wang et al. (2022). Frontiers in microbiology, 13, S. 910343; Sadremomtaz et al. (2022). Journal of medicinal chemistry, 4, S. 2836-2847) were not very successful, the inventors anticipated that some sequences in the RBD recognized by neutralizing antibodies in response to SARS-CoV-2 infection or vaccination should be able to inhibit RBD-ACE-2 complex formation. An early epitope study of SARS-CoV-2 proteins using a polypeptide microarray identified sequences 351 to 364 and 452 to 465 of the RBD as neutralizing epitopes (Wang et al. (2020). ACS Cent. Sci., 12, S. 2238-2249). Sequence alignments of the common alpha, beta, gamma, and delta (kappa) VOCs suggest that most mutations occur in the investigated regions 392 to 421, 452 to 465, and 481 to 510 of the RBD, most likely allowing the VOCs to (partially) escape the immune response of vaccinated or infected individuals. However, these sequences do not represent universal epitopes that would allow the identification of IgG antibodies better suited to neutralize emerging variants of the virus (Schwarze et al. (2022). Pathogens, 12, S. 1515).

The inventors have tested four polypeptide families corresponding to residues 351 to 364, 392 to 421, 452 to 465, and 481 to 510 of the RBD sequence, including relevant mutations of important SARS-CoV-2 VOCs until the end of summer of 2024, namely alpha, beta, gamma, delta (kappa), and omicron variants BA.1 to BA.5, XBB, and KP.3 for their inhibitory effects. The polypeptide families were selected based on (i) reported interaction sites between the RBD and the ACE-2 receptor (Jawad et al. (2021). Journal of chemical information and modeling, 9, S. 4425-4441), (ii) previously evaluated polypeptides (Mahindra et al. (2021). PloS one, 11, e0260283), and (iii) interaction sites predicted by modeling approaches (Jawad et al. (2021). Journal of chemical information and modeling, 9, S. 4425-4441; Lan et al. (2020). Nature, 7807, S. 215-220) **(Table 1).** Considering all mutations of the most common VOCs of SARS-CoV-2 identified in Europe in 2021 and 2022, the inventors synthesized up to seven VOC-specific RBD polypeptides per sequence, all of which were obtained in high purities **(Table 1,** **Figure 1****).** In a previous study, the inventors demonstrated that some of these polypeptides are recognized by antibodies present in the serum of patients infected with SARS-CoV-2 (Schwarze et al. (2022). Pathogens, 12, S. 1515). However, the established polypeptide-based ELISAs did not provide a diagnostic advantage over the RBD- and S1-based ELISAs.

**Table 1 - Synthetic RBD polypeptides used in this study. The mutation sites of the different VOCs are highlighted in bold and underlined in the sequences. All polypeptides were obtained as C-terminal amides.**

| **Polypeptide** | **Polypeptide sequence** | **Mutation s** | **Variants** | **SEQ ID NO:** |
|---|---|---|---|---|
| | **RBD 351-364** | | | |
| p351wt | AWNRKRISNCVAD | none | WT, alpha, beta, gamma, delta (kappa) BA, BQ, XBB | 3 |

| | **RBD 392-421** | | | |
|---|---|---|---|---|
| p392wt | FTNVYADSFVIRGDEVRQIAPGQTGKIADY | none | WT, alpha, delta (kappa) | 4 |
| p392b | FTNVYADSFVIRGDEVRQIAPGQTG**N**IADY | K417N | beta, BA.1 | 5 |
| p392g | FTNVYADSFVIRGDEVRQIAPGQTG**T**IADY | K417T | gamma | 6 |
| p392BA.2 | FTNVYADSFVIRG**N**EV**S**QIAPGQTG**N**IADY | D405N, R408S, K417N | BA.2-5, BQ.1, XBB, KP.3 | 7 |
| p392BA.2T | FTNVYADSFVIRG**N**EV**S**QIAPGQTG**T**IADY | D405N, R408S, K417T | BA.2,3, BQ (K417T) | 8 |

| | **RBD 452-465** | | | |
|---|---|---|---|---|
| p452wt | LYRLFRKSNLKPFE | none | WT | 9 |
| p452d | **R**YRLFRKSNLKPFE | L452R | delta (kappa), BA.4-5 | 10 |

| | **RBD 481-510** | | | |
|---|---|---|---|---|
| p481wt | NGVEGFNCYFPLQSYGFQPTNGVGYQPYRV | none | WT | 11 |
| p481a | NGVEGFNCYFPLQSYGFQPT**Y**GVGYQPYRV | N501Y | alpha | 12 |
| p481b | NGV**K**GFNCYFPLQSYGFQPT**Y**GVGYQPYRV | K484K, N501Y | beta, gamma | 13 |
| p481d | NGV**Q**GFNCYFPLQSYGFQPTNGVGYQPYRV | K484Q | delta (kappa) | 14 |
| p481BA.1 | NGV**A**GFNCYFPL**K**SY**S**F**R**PT**Y**GVG**H**QPYRV | K484A, Q493K, G496S, Q498R, N501Y, Y505H | BA.1 | 15 |
| p481BA.4 | NGV**A**G**V**NCYFPLQSYGF**R**PT**Y**GVG**H**QPYRV | K484A, F486V, Q498R, Y505H | BA.4,5, BQ.1 | 16 |
| p481F497V | NGV**A**G**V**NCYFPLQSYG**VR**PT**Y**GVG**H**QPYRV | K484A, F486V, F497V, Q498R, N501Y, Y505H | BA (F497V) | 17 |

| | **N-protein 66-79** | | | |
|---|---|---|---|---|
| pNP66 | KEDLKFPRGQGVPI | none | WT | 18 |

### Example 3 - ACE-2 inhibition assay and binding studies for wild-type RBD

All 15 RBD-derived polypeptides (see **Table 1)** were tested for inhibition in a recently reported *in vitro* ACE-2 inhibition assay (see **Example 1-E,** *supra,* and (Schwarze et al. (2022). Frontiers in immunology, 13, S. 915034)), which assesses binding of the ACE-2 receptor in solution to coated RBD in the presence of substances that potentially inhibit the interaction **(****Figure 2****).** The short polypeptides corresponding to RBD sequences 351 to 364 and 452 to 465 did not interfere with binding in the concentration range tested. The alpha variant of RBD sequence 481 to 510 and all variants of RBD sequence 392 to 421 inhibited the interaction with normalized IC₅₀ values ranging from 4.9 ± 2.3 µmol/L (p481a) to 46.1 ± 10.0 µmol/L (p392BA.2T), which was more than 100-fold weaker than the IC₅₀ of 29 nmol/L of recombinant full-length wild-type RBD **(****Figure 1 to 5****).** Interestingly, neither the selected N-polypeptide nor the antimicrobial polypeptide LL-37, previously reported to inhibit RBD-ACE-2 receptor binding (Saini et al. (2022). Microbiological research, 265, S. 127206; Nireeksha et al. (2022). European journal of dentistry, 3, S. 478-487), affected RBD binding **(****Figures 3 to 4****).**

Apart from p481a, where the IC₅₀ value varied widely between assays, the p392wt polypeptide reproducibly showed a low IC₅₀ (6.4 µmol/L) using the wild-type RBD as a binding competitor. Surprisingly, all mutated sequences of the wild-type p392 sequence showed a higher IC₅₀ **(****Figure 2****),** suggesting that the escape mutations reduce ACE-2 receptor binding, at least at the polypeptide level, which could be due to different or more flexible polypeptide conformations. It should be noted that the K417T mutation (p392BA.2T) was studied in addition to the K417N mutation (p392BA.2) for the omicron variants, because the polypeptide p392g of the gamma variant (K417T) showed a stronger inhibitory effect (IC₅₀ = 30.56 µmol/L) than the polypeptide p392b (IC₅₀ = 40.16 µmol/L) representing the beta variant (K417N).

In addition to the inhibition assay, the inventors also examined polypeptide binding to the ACE-2 receptor by MST using a fluorophore complexed to the His-tag of the ACE-2 receptor (see **Example1-*****F,** supra,* and (NanoTemper Technologies. Inc. (2019))). Consistent with the inhibition assay, no binding was observed for the short RBD polypeptides (351 to 364 and 452 to 465) and the control N-polypeptide **(****Figure 5****).** All p481 polypeptides bound well, but it turned out that they bound also to the His6 control polypeptide provided with the Monolith His-Tag Labeling Kit **(****Figure 6****).** This suggests that this sequence competes with the fluorophore for binding to His-tagged ACE-2 and thus cannot be assayed with the MST setup used, although K_{d} values of ~10 nmol/L to 540 nmol/L have been reported for ACE-2-based sequences binding to wild-type RBM using a similar MST approach (Sadremomtaz et al. (2022). Journal of medicinal chemistry, 4, S. 2836-2847). All p392 polypeptides bound to the ACE-2 receptor, but not to the His-tag control polypeptide, with K_{d} values ranging from 8.5 µmol/L for the wild-type sequence, which was only slightly worse than the RBD protein (K_{d} = 4.95 µmol/L), to 49.7 µmol/L for p392g **(****Figure 2** and **Figures 5** to 6).

It is evident that the best binding polypeptide p392wt (K_{d} = 8.50 ± 3.16 µmol/L) provided the strongest inhibitory effect (IC₅₀ = 6.40 ± 0.61 µmol/L), while the mutant p392 variants showed similarly higher K_{d} and IC₅₀ values in the range of 26.6 to 49.7 µmol/L This clearly indicated a correlation between the K_{d} of the polypeptide-ACE-2 binding and the inhibition of RBD-ACE-2 receptor binding represented by the IC₅₀.

Using an RBD-ACE-2 binding assay with the recombinant RBD coated on a microtiter plate, the inventors unexpectedly observed very good inhibition of up to 96% (see, for example, **Figure 4****).** The polypeptide p392BA.2, which resembles residues 392 to 421 of the omicron variants BA.2-5, BQ.1, XBB, KP.3 showed the strongest effect against all VOCs tested with an average IC₅₀ of 18.33 µmol/L (see **Example 4,** below). Interestingly, this sequence is conserved in the omicron BA.2 variant and may therefore have an inhibitory effect against more recent VOCs including XBB, KP.3 and future SARS-CoV-2 variants (Enabled by data from GISAID). The potential therapeutic effect was confirmed in a pseudo-virus assay using HEK cells expressing ACE-2 (see **Example 5,** below).

### Example 4 - ACE-2 inhibition assay of VOC RBDs

The inventors extended the RBD-ACE-2 inhibition assay to six VOCs, including two omicron variants, expressed in HEK cells (see **Example 1-B)** and coated on the microtiter plate (see **Example 1-E) (****Figure 7 to 9****).** The polypeptide pNP66 (negative control) did not inhibit complex formation, whereas the full-length RBD variants (positive control) effectively inhibited ACE-2 receptor binding to the corresponding coated RBD variant with IC₅₀ values ranging from 3.65 ± 0.63 nmol/L (BA.4/5) to 28.58 ± 7.56 nmol/L (wild-type). Since RBD variant BA.1 was not available in sufficient quantities, the assay was incubated with wild-type RBD, which did not inhibit binding.

Notably, the inhibition of ACE-2 receptor binding was dependent on the specific mutant polypeptide and RBD variant. Overall, the wild-type and BA.4/5 RBDs were most significantly inhibited by all mutant polypeptides, often with greater inhibition than the other RBD variants by their respective p392 polypeptide (see e.g., **Figure 10****).** The p392wt polypeptide slightly inhibited the recognition of the alpha, beta, and gamma VOCs, suggesting a potentially stronger binding between these VOCs and the ACE-2 receptor, or a distinct binding mode or contact area. Unexpectedly, the RBDs of the later emerging delta and omicron VOCs were more effectively suppressed by p392 polypeptides than the earlier VOCs, regardless of the mutation site. This pattern was also observed for the p392wt polypeptide, except for the omicron BA.1 variant. Among all polypeptides, p392BA.2 showed the most potent inhibition against the RBDs of omicron BA.1 (IC₅₀ = 4.74 ± 1.56 µmol/L) and omicron BA.4 (IC₅₀ = 4.76 ± 1.94 µmol/L), which is evident from the lowest IC₅₀ values. Polypeptide p392BA.2 was the most potent inhibitor against all tested variants with an average IC₅₀ of 18.33 µmol/L. Furthermore, it effectively suppressed the binding of the relevant omicron variants. It is important to note that the RBD sequence 392 to 421 is highly conserved in all variants after BA.2. Therefore, it is expected that p392BA.2 will also inhibit the later omicron variants XBB, BQ.1, and KP.3. The polypeptide p481a was also investigated because it reasonably inhibited the binding of the wild-type RBD with an IC₅₀ of 4.92 ± 2.33 µmol/L. However, it did not inhibit the binding of any of the other RBD variants.

Overall, all p392 polypeptides evaluated demonstrated good inhibition against omicron variant BA.4/5, which is an encouraging result for potential therapeutic applications. Although the inhibitory effect of the p392 polypeptides was affected by mutation sites in the p392 sequence of the RBD variants, all polypeptides still produced efficacies ranging from 50% to 96% against all RBDs and even efficacies greater than 90% against the RBD BA.4 variant. Consistently, the p392BA.2 polypeptide was identified as the most potent inhibitor (96%) against the gamma RBD variant **(****Figure 7****).**

### Example 5 - Pseudovirus Assay

The inhibitory effect of the polypeptides quantified *in vitro* (see **Example 4,** *supra)* was extended to a commercial pseudovirus kit based on HEK293 cells overexpressing ACE-2 (see **Example 1-H,** *supra),* which can be infected with a SARS-CoV-2 pseudovirus encoding the wild-type S protein (Abnova Corporation (2023)). Although the pseudovirus contained only the wild-type RBD, all p392 polypeptides inhibited infection of HEK293 cells **(****Figures 11****).** The measured relative luminescence units (RLUs) were normalized to the background (0%, empty well) and to the cells incubated only with the pseudovirus (100%). The control was an anti-SARS-CoV-2 antibody, which quantitatively inhibits cell infection at a concentration of 1 mg/L (0%) (Abnova Corporation (2023)). Similar to the *in vitro* RBD-ACE-2 inhibition assay (see **Example 4,** *supra),* polypeptides p481a and pNP66 did not inhibit the pseudovirus. All p392 polypeptides, except p392BA.2, reduced the pseudovirus infection rate to 58% to 71% at a polypeptide concentration of 400 mg/L, the highest polypeptide concentration used in the *in vitro* assay. At the highest polypeptide concentration tested (800 mg/L) all p392 polypeptides reduced the pseudovirus infection rate further to 1.2% to 55%, with p392wt being the most potent polypeptide, consistent with the *in vitro* assay **(****Figure 2****).**

Unexpectedly, the wild-type RBD expressed in HEK293S GnTI- cells showed pseudovirus infection rates of only 68% (100 mg/L) to 50% (200 mg/L), which is much worse than in the *in vitro assay.* However, it should be noted that the fourfold lower protein concentration corresponds to an approximately 40-fold lower molar protein concentration of ~6.8 µmol/L compared to ~244 µmol/L for the polypeptide **(****Figure 12****).** Furthermore, the weak effect of the RBD protein in the pseudovirus assay (max. 50%, **Figure 11****),** seen at a two-fold higher concentration than in the ACE-2 binding assay **(****Figure 3****),** is most likely related to its monomeric state compared to the S protein trimer in the native spike protein. Other studies have shown that homotrimers and homotetramers are more potent and more similar to the viral spike protein (Basavarajappa et al. (2022). iScience, 8, S. 104716; Liu et al. (2022). Frontiers in immunology, 13, S. 960094).

The p392wt polypeptide was an effective binding inhibitor in the pseudovirus assay **(****Figure 11****),** as efficient as monoclonal anti-SARS-CoV-2 antibodies. The inhibitory effect of the polypeptides is further emphasized by the fact that ACE-2 was overexpressed on the surface of HEK cells and was therefore present in higher numbers than in natural cells, making them more susceptible to viral infection than cells in the human body. The higher infectivity rate (corresponding to higher relative luminescence units (RLU)) of the virus at medium polypeptide concentrations (10.7 to 400 mg/L) compared to DMEM as control is associated with an up to fivefold increase in cell growth, as more cells are available for infection (see also **Example 6** and **Figure 13****).** Due to the higher cell counts, polypeptides are even more effective at their inhibition concentration. This explains also the relatively low nominal inhibition of p392g **(****Figure 11****),** as cell growth was the highest in its presence **(****Figure 13****).**

### Example 6 - Cytotoxicity assay

Since the standard MTT (3-(4,5-Dimethylthiazol-2-yl)-2,5-diphenyltetrazoliumbromid) method (Brakel et al. (2022). Frontiers in chemistry, S. 798006) showed high inter-assay variability (data not shown), cell viability was assessed using the CASY cell counting method, which combines electronic current exclusion and pulse field analysis (see **Example 1-I,** *supra,* and (cytena GmbH)). This method has been shown to provide very sensitive IC₅₀ values for 50 toxins that were only slightly affected by other substances (Lindl et al. (2005). Alternatives to laboratory animals: ATLA, 6, S. 591-601). All p392 polypeptides were non-toxic to HEK293 cells at a polypeptide concentration of 800 mg/L **(****Figure 13****),** whereas p481a was toxic reducing cell viability to about one third similar to 12% DMSO. Even RBDwt was slightly toxic (cell count reduced by 20%) at a protein concentration of 200 mg/L. Interestingly, all p392 polypeptides increased viable cell counts by at least 25%, with p392g increasing viability sixfold. The best pseudovirus inhibitory polypeptide, p392wt, was further tested at the concentration showing the highest infection rate (221 mg/L, **Figure 13b****).** After 48 h, only a very weak cytotoxic effect was observed in the absence of pseudovirus compared to DMEM medium as control, while the polypeptide protected the cells against pseudovirus as indicated by an approximately four-fold higher cell viability compared to the control without polypeptide **(****Figure 13b****).**

### Example 7 - Influence of polypeptides on IgG antibodies induced via vaccination by ELISA

Having confirmed the protective effect of the p391 polypeptides on HEK293 cells against pseudovirus infection (see **Example 4,** *supra),* the question remained whether these RBD-derived polypeptides would bind to anti-S1 antibodies induced by vaccination and thus inhibit these antibodies, or whether they would have synergistic effects. When serum samples from vaccinated individuals (see **Example 1-A,** *supra)* were spiked with a serial dilution of the polypeptides, neither the negative control polypeptide pNP66 nor any of the RBD-polypeptides reduced the readout, even at the highest concentrations tested of 400 mg/L (depending on the polypeptide mass 116 to 261 µmol/L; **Figure 12a****),** in a previously reported IgG ELISA (see Example 1-D, supra, and (Schwarze et al. (2022). Frontiers in immunology, 13, S. 915034)). The full-length RBD protein reduced the readout over the concentration range tested from 100 mg/L (3.42 µmol/L) to 0.2 µg/L (6.68 pmol/L), confirming the validity of the assay **(****Figure 12b****).**

### Example 8 - Serum stability assay

Since degradation of the polypeptides by proteases present in the serum during the ELISA assay (see **Example 7,** *supra)* could not be excluded, the protease stability of the polypeptides was tested by incubation in human serum (see **Example 1-A,** *supra)* under the same conditions as in the ELISA (serum 100-fold diluted in PBS), except for a longer incubation time of 90 min instead of 60 min (compare **Example 1-B and 1-G,** *supra).* The polypeptide serum stability assay used a polypeptide concentration of 0.6 g/L, whereas the IgG ELISA to test for polypeptide interference with antibodies in sera from vaccinated patients (see **Example 7,** **Figure 11****)** used much lower polypeptide concentrations ranging from 780 ng/L to 0.4 g/L (see **Examples 1-B,** *supra).* However, the ELISA used serum diluted 500-fold, i.e., 0.2 µL of serum per well compared to 1 µL of serum per well in the stability assay. Therefore, it is highly probable that the polypeptides are more stable in the ELISA than indicated by the half-live times in serum. The polypeptide concentration in the serum stability assay (0.12 g/L; 35-78 µmol/L) is within the range tested for inhibitory effect and close to the IC₅₀ (~26 µmol/L, **Figure 7****),** indicating surprisingly good *in vivo* activity.

Polypeptides p481d and p481BA.1 were the most rapidly degraded, with only ~40% of the original quantity remaining after 90 min, while all p392 polypeptides were virtually not degraded **(Figure 15).** The most likely explanation for the observed increase in polypeptide amount (of any of the tested polypeptides, in particular p392 polypeptides) is that the polypeptides dissolve better at 37°C after a certain time at the respective concentration (600 mg/L) and therefore, after a certain incubation time, it appears that more polypeptides are present in the respective aliquots. However, since the aliquots of all three-time samples originated from one respective stock solution an actual increase in polypeptide amount can be ruled out. Therefore, the p392 polypeptides should be stable in the ELISA, suggesting that the polypeptides do, indeed, not inhibit antibody-RBD binding **(****Figure 12****).**

Concluding, the unexpectedly most promising polypeptides were the p392 polypeptides covering residues 392-421 of the RBD, which contains only one previously reported strong binding site, K417, which forms a salt bridge to D30 of ACE-2 at the protein level and was mutated twice in VOCs. Surprisingly, all p392 polypeptides yielded good to moderate K_{d} values for ACE-2 binding and similarly good to moderate IC₅₀ values for inhibition of the RBD-ACE-2 interaction in the range of 8.5 to 50 µmol/L for the wild-type and gamma RBD sequences, respectively. Interestingly, the binding constant of p392wt is only ~1,7-fold higher than that of the wild-type RBD (K_{d} = 4.95 µmol/L), which contains all known strong and weak binding sites and contains 222 residues compared to 30 residues in the polypeptide. Based on the reported binding sites of RBD and ACE-2 (Jawad et al. (2021). Journal of chemical information and modeling, 9, S. 4425-4441; Lan et al. (2020). Nature, 7807, S. 215-220), it can be speculated that the p392 polypeptides also form this salt bridge with D30 of ACE-2 via K26 (K417 in wild-type RBD). Thus, the polypeptide would block possibly nearby binding sites T27, E35, and Q42, all located on the α1-helix of ACE-2 **(****Figure 1****).**

However, p392wt was least efficient in inhibiting ACE-2 binding of RBD variants alpha, beta, and BA.1 **(****Figure 7****).** Considering the inhibitory effects on all RBDs studied, p392BA.2 was the best with IC₅₀ values ranging from 4.7 µmol/L (BA.1 and BA.4/5) to 24.7 µmol/L (alpha) for the VOCs compared to only ~44 µmol/L for the wild-type RBD, which was similar or up to three times worse than the full-length wild-type RBD.

The p392 polypeptides inhibited the binding of wild-type RBD to ACE-2 with promising IC₅₀ values in the low micromolar range, but the wild-type RBD protein was more than 200-fold more potent with an IC₅₀ of ~29 nmol/L, which can be attributed to the much larger contact area covering the strong and weak interaction sites mentioned above. This inhibitory effect of the RBD monomer has been reported previously (Basavarajappa et al. (2022). iScience, 8, S. 104716; Liu et al. (2022). Frontiers in immunology, 13, S. 960094). The very good IC₅₀ suggests strong inhibition of ACE-2 recognition of SARS-CoV, but the RBD is also recognized by anti-RBD antibodies, virtually depleting the mucosa or blood for neutralizing IgA or IgG antibodies, respectively. In this respect, treatment with an RBD protein could favor SARS-CoV infection by blocking neutralizing antibodies after treatment, while antibody binding would also block the contact area of the administered RBD proteins supposed to bind to ACE-2. Indeed wild-type RBD suppressed antibody recognition of wild-type RBD in serum samples **(****Figure 12****).** In contrast, none of the RBD-derived polypeptides added to serum suppressed the signal in the ELISA indicating that the p392 would not inhibit neutralizing antibodies and thus could act synergistically with antibodies to prevent SARS-CoV infection. Since the p392 polypeptides were not toxic to HEK293 cells, they are valid lead structures for the development of compounds to prevent SARS-CoV infection, for example by, but not limited to, application as a nasal spray or aerosol inhalator.

### Sequence Listing

| **SEQ ID NO** | **Description** | **Sequence** |
|---|---|---|
| 1 | Full length S-protein | |
| 2 | Full length RBD (319-541), RBDwt | RVQPTESIVRFPNITNLCPFGEVFNATRFASVYAWNRKRISNCVADYSVLYNSASF STFKCYGVSPTKLNDLCFTNVYADSFVIRGDEVRQIAPGQTGKIADYNYKLPDDF TGCVIAWNSNNLDSKVGGNYNYLYRLFRKSNLKPFERDISTEIYQAGSTPCNGVE GFNCYFPLQSYGFQPTNGVGYQPYRVVVLSFELLHAPATVCGPKKSTNLVKNKCV NF |
| 3 | p351wt | AWNRKRISNCVAD |
| 4 | p392wt | FTNVYADSFVIRGDEVRQIAPGQTGKIADY |
| 5 | p392b | FTNVYADSFVIRGDEVRQIAPGQTG**N**IADY |
| 6 | p392g | FTNVYADSFVIRGDEVRQIAPGQTG**T**IADY |
| 7 | p392BA.2 | FTNVYADSFVIRG**N**EV**S**QIAPGQTG**N**IADY |
| 8 | p392BA.2T | FTNVYADSFVIRG**N**EV**S**QIAPGQTG**T**IADY |
| 9 | p452wt | LYRLFRKSNLKPFE |
| 10 | p452d | **R**YRLFRKSNLKPFE |
| 11 | p481wt | NGVEGFNCYFPLQSYGFQPTNGVGYQPYRV |
| 12 | p481a | NGVEGFNCYFPLQSYGFQPT**Y**GVGYQPYRV |
| 13 | p481b | NGV**K**GFNCYFPLQSYGFQPT**Y**GVGYQPYRV |
| 14 | p481d | NGV**Q**GFNCYFPLQSYGFQPTNGVGYQPYRV |
| 15 | p481BA.1 | NGV**A**GFNCYFPL**K**SY**S**F**R**PT**Y**GVG**H**QPYRV |
| 16 | p481BA.4 | NGV**A**G**V**NCYFPLQSYGF**R**PT**Y**GVG**H**QPYRV |
| 17 | p481F497V | NGV**A**G**V**NCYFPLQSYG**VR**PT**Y**GVG**H**QPYRV |
| 18 | pNP66 | KEDLKFPRGQGVPI |
| 19 | LL-37 | LLGDFFRKSKEKIGKEFKRIVQRIKDFLRNLVPRTES |

### References

Abe, Kento T.; Li, Zhijie; Samson, Reuben; Samavarchi-Tehrani, Payman; Valcourt, Emelissa J.; Wood, Heidi et al. (2020): A simple protein-based surrogate neutralization assay for SARS-CoV-2. In: JCI insight 5 (19). DOI: 10.1172/jci.insight.142362.
Abnova Corporation (2023): www.abnova.com COVID-19 Pseudovirus Neutralizing Antibody Assay (Luciferase). Catalog Number KA6152, Version: 1.3. Online available:
   https://www.abnova.com/upload/media/product/protocol_pdf/KA6152.pdf, last checked on 23.01.2024.
Akash, Shopnil; Khan, Md Shajib; Mukerjee, Nobendu; Bibi, Shabana (2023): Re-emergence of SARS-CoV-2 Omicron subvariant XBB.1.5: present status, treatment, and future outlook - 2023. In: International Journal of Surgery (London, England) 109 (4), S. 658-659. DOI: 10.1097/JS9.0000000000000258.
Aricescu, A. Radu; Lu, Weixian; Jones, E. Yvonne (2006): A time- and cost-efficient system for high-level protein production in mammalian cells. In: Acta crystallographica 62 (Pt 10), S. 1243-1250. DOI: 10.1107/S0907444906029799.
Basavarajappa, Shrikanth C.; Liu, Angela Rose; Bruchez, Anna; Li, Zhenlu; Suzart, Vinicius G.; Liu, Zhonghua et al. (2022): Trimeric receptor-binding domain of SARS-CoV-2 acts as a potent inhibitor of ACE2 receptor-mediated viral entry. In: iScience 25 (8), S. 104716. DOI: 10.1016/j.isci.2022.104716.
Brakel, Alexandra; Kolano, Lisa; Kraus, Carl N.; Otvos, Laszlo; Hoffmann, Ralf (2022): Functional Effects of ARV-1502 Analogs Against Bacterial Hsp70 and Implications for Antimicrobial Activity. In: Frontiers in chemistry 10, S. 798006. DOI: 10.3389/fchem.2022.798006.
Chitsike, Lennox; Krstenansky, John; Duerksen-Hughes, Penelope J. (2021): ACE2: S1 RBD Interaction-Targeted Peptides and Small Molecules as Potential COVID-19 Therapeutics. In: Advances in pharmacological and pharmaceutical sciences 2021, S. 1828792. DOI: 10.1155/2021/1828792.
cytena GmbH: CASY CELL COUNTER & ANALYZER. Online available: https://www.cytena.com/wp-content/uploads/2020/10/CASY-brochure-digital_201021_LR.pdf.
Enabled by data from GISAID: covSPECTRUM. Online available: https://covspectrum.org/explore/Germany/AllSamples/Past6M, last checked on 08.01.2024.
Gottlieb, Michael; Spatz, Erica S.; Yu, Huihui; Wisk, Lauren E.; Elmore, Joann G.; Gentile, Nicole L. et al. (2023): Long COVID Clinical Phenotypes up to 6 Months After Infection Identified by Latent Class Analysis of Self-Reported Symptoms. In: Open forum infectious diseases 10 (7), ofad277. DOI: 10.1093/ofid/ofad277.
Han, Jin H.; Womack, Kelsey N.; Tenforde, Mark W.; Files, D. Clark; Gibbs, Kevin W.; Shapiro, Nathan I. et al. (2022): Associations between persistent symptoms after mild COVID-19 and long-term health status, quality of life, and psychological distress. In: Influenza and other respiratory viruses 16 (4), S. 680-689. DOI: 10.1111/irv.12980.
Huang, Chaolin; Wang, Yeming; Li, Xingwang; Ren, Lili; Zhao, Jianping; Hu, Yi et al. (2020): Clinical features of patients infected with 2019 novel coronavirus in Wuhan, China. In: The Lancet 395 (10223), S. 497-506. DOI: 10.1016/S0140-6736(20)30183-5.
Jawad, Bahaa; Adhikari, Puja; Podgornik, Rudolf; Ching, Wai-Yim (2021): Key Interacting Residues between RBD of SARS-CoV-2 and ACE2 Receptor: Combination of Molecular Dynamics Simulation and Density Functional Calculation. In: Journal of chemical information and modeling 61 (9), S. 4425-4441. DOI: 10.1021/acs.jcim.1c00560.
Khater, Ibrahim; Nassar, Aaya (2022): Potential antiviral peptides targeting the SARS-CoV-2 spike protein. In: BMC pharmacology & toxicology 23 (1), S. 91. DOI: 10.1186/s40360-022-00627-w.
Kim, Seonghan; Liu, Yi; Ziarnik, Matthew; Cao, Yiwei; Zhang, X. Frank; Im, Wonpil (2022): Binding of Human ACE2 and RBD of Omicron Enhanced by Unique Interaction Patterns Among SARS-CoV-2 Variants of Concern. In: biorxiv : the preprint server for biology. DOI: 10.1101/2022.01.24.477633.
Lan, Jun; Ge, Jiwan; Yu, Jinfang; Shan, Sisi; Zhou, Huan; Fan, Shilong et al. (2020): Structure of the SARS-CoV-2 spike receptor-binding domain bound to the ACE2 receptor. In: Nature 581 (7807), S. 215-220. DOI: 10.1038/s41586-020-2180-5.
Lindl, Toni; Lewandowski, Birgit; Schreyögg, Sonya; Stäudte, Andrea (2005): An evaluation of the in vitro cytotoxicities of 50 chemicals by using an electrical current exclusion method versus the neutral red uptake and MTT assays. In: Alternatives to laboratory animals: ATLA 33 (6), S. 591-601. DOI: 10.1177/026119290503300614.
Liu, Kui; Fang, Yuan-Yuan; Deng, Yan; Liu, Wei; Wang, Mei-Fang; Ma, Jing-Ping et al. (2020): Clinical characteristics of novel coronavirus cases in tertiary hospitals in Hubei Province. In: Chinese Medical Journal 133 (9), S. 1025-1031. DOI: 10.1097/CM9.0000000000000744.
Liu, Zheng; Yang, Chenglu; Zhang, Haokun; Cao, Guojie; Wang, Senzhen; Yin, Siwen; Wang, Yanming (2022): SARS-CoV-2 tetrameric RBD protein blocks viral infection and induces potent neutralizing antibody response. In: Frontiers in immunology 13, S. 960094. DOI: 10.3389/fimmu.2022.960094.
Mahindra, Amit; Tejeda, Gonzalo; Rossi, Mario; Janha, Omar; Herbert, Imogen; Morris, Caroline et al. (2021): Peptides derived from the SARS-CoV-2 receptor binding motif bind to ACE2 but do not block ACE2-mediated host cell entry or pro-inflammatory cytokine induction. In: PloS one 16 (11), e0260283. DOI: 10.1371/journal.pone.0260283.
NanoTemper Technologies. Inc. (2019): MO-L018 Monolith His-Tag Labeling Kit RED-tris-NTA 2nd Generation. Manuals. Online available: https://support.nanotempertech.com/hc/en-us/articles/18013589922193-MO-L018-Monolith-His-Tag-Labeling-Kit-RED-tris-NTA-2nd-Generation, last checked on 23.01.24.
Nireeksha, Nireeksha; Gollapalli, Pavan; Varma, Sudhir Rama; Hegde, Mithra N.; Kumari, N. Suchetha (2022): Utilizing the Potential of Antimicrobial Peptide LL-37 for Combating SARS-COV- 2 Viral Load in Saliva: an In Silico Analysis. In: European journal of dentistry 16 (3), S. 478-487. DOI: 10.1055/s-0041-1739444.
O'Laughlin, Kelli N.; Thompson, Matthew; Hota, Bala; Gottlieb, Michael; Plumb, Ian D.; Chang, Anna Marie et al. (2022): Study protocol for the Innovative Support for Patients with SARS-COV-2 Infections Registry (INSPIRE): A longitudinal study of the medium and long-term sequelae of SARS-CoV-2 infection. In: PloS one 17 (3), e0264260. DOI: 10.1371/journal.pone.0264260.
Rajpoot, Sajjan; Ohishi, Tomokazu; Kumar, Ashutosh; Pan, Qiuwei; Banerjee, Sreeparna; Zhang, Kam Y. J.; Baig, Mirza S. (2021): A Novel Therapeutic Peptide Blocks SARS-CoV-2 Spike Protein Binding with Host Cell ACE2 Receptor. In: Drugs in R&D 21 (3), S. 273-283. DOI: 10.1007/s40268-021-00357-0.
Rathod, Shravan B.; Prajapati, Pravin B.; Punjabi, Lata B.; Prajapati, Kuntal N.; Chauhan, Neha; Mansuri, Mohmedyasin F. (2020): Peptide modelling and screening against human ACE2 and spike glycoprotein RBD of SARS-CoV-2. In: In silico pharmacology 8 (1), S. 3. DOI: 10.1007/s40203-020-00055-w.
Sadremomtaz, Afsaneh; Al-Dahmani, Zayana M.; Ruiz-Moreno, Angel J.; Monti, Alessandra; Wang, Chao; Azad, Taha et al. (2022): Synthetic Peptides That Antagonize the Angiotensin-Converting Enzyme-2 (ACE-2) Interaction with SARS-CoV-2 Receptor Binding Spike Protein. In: Journal of medicinal chemistry 65 (4), S. 2836-2847. DOI: 10.1021/acs.jmedchem.1c00477.
Saini, Jasleen; Kaur, Pritpal; Malik, Naveen; Lakhawat, Sudarshan Singh; Sharma, Pushpender Kumar (2022): Antimicrobial peptides: A promising tool to combat multidrug resistance in SARS CoV2 era. In: Microbiological research 265, S. 127206. DOI: 10.1016/j.micres.2022.127206.
Schwarze, Mandy; Krizsan, Andor; Brakel, Alexandra; Pohl, Fabian; Volke, Daniela; Hoffmann, Ralf (2022): Cross-Reactivity of IgG Antibodies and Virus Neutralization in mRNA-Vaccinated People Against Wild-Type SARS-CoV-2 and the Five Most Common SARS-CoV-2 Variants of Concern. In: Frontiers in immunology 13, S. 915034. DOI: 10.3389/fimmu.2022.915034.
Schwarze, Mandy; Luo, Ji; Brakel, Alexandra; Krizsan, Andor; Lakowa, Nicole; Grünewald, Thomas et al. (2022): Evaluation of S- and M-Proteins Expressed in Escherichia coli and HEK Cells for Serological Detection of Antibodies in Response to SARS-CoV-2 Infections and mRNA-Based Vaccinations. In: Pathogens 11 (12), S. 1515. DOI: 10.3390/pathogens11121515.
Tallei, Trina Ekawati; Alhumaid, Saad; AlMusa, Zainab; Fatimawali; Kusumawaty, Diah; Alynbiawi, Ahlam et al. (2023): Update on the omicron sub-variants BA.4 and BA.5. In: Reviews in medical virology 33 (1), e2391. DOI: 10.1002/rmv.2391.
van Vo, Giau; Bagyinszky, Eva; An, Seong Soo A. (2022): COVID-19 Genetic Variants and Their Potential Impact in Vaccine Development. In: Microorganisms 10 (3). DOI: 10.3390/microorganisms10030598.
Wang, Cheng; Wang, Shaobo; Li, Daixi; Chen, Peiqin; Han, Songling; Zhao, Gaomei et al. (2021): Human Cathelicidin Inhibits SARS-CoV-2 Infection: Killing Two Birds with One Stone. In: ACS infectious diseases 7 (6), S. 1545-1554. DOI: 10.1021/acsinfecdis.1c00096.
Wang, Dawei; Hu, Bo; Hu, Chang; Zhu, Fangfang; Liu, Xing; Zhang, Jing et al. (2020): Clinical Characteristics of 138 Hospitalized Patients With 2019 Novel Coronavirus-Infected Pneumonia in Wuhan, China. In: JAMA 323 (11), S. 1061. DOI: 10.1001/jama.2020.1585.
Wang, Hongye; Wu, Xian; Zhang, Xiaomei; Hou, Xin; Liang, Te; Wang, Dan et al. (2020): SARS-CoV-2 Proteome Microarray for Mapping COVID-19 Antibody Interactions at Amino Acid Resolution. In: ACS Cent. Sci. 6 (12), S. 2238-2249. DOI: 10.1021/acscentsci.0c00742.
Wang, Ting; Xu, Jie; Wang, Beibei; Wang, Yulian; Zhao, Wei; Xiang, Bin et al. (2022): Receptor-binding domain-anchored peptides block binding of severe acute respiratory syndrome coronavirus 2 spike proteins with cell surface angiotensin-converting enzyme 2. In: Frontiers in microbiology 13, S. 910343. DOI: 10.3389/fmicb.2022.910343.

All references cited herein above are fully incorporated by reference.

Having now fully described the invention, it will be understood by a person skilled in the art that the invention may be practiced within a wide and equivalent range of conditions, parameters and the like, without affecting the spirit or scope of the invention or any embodiment thereof.

## Claims

1. A polypeptide for use in treatment and/or prevention of coronavirus infection, comprising an amino acid sequence according to SEQ ID NO: 4 or an amino acid sequence of at least 80% sequence identity to SEQ ID NO: 4.

2. The polypeptide for use according to claim 1, comprising amino acid substitutions relative to SEQ ID NO: 4 selected from:
(i) K26N,
(ii) K26T,
(iii) D14N, R17S, K26N, or
(iv) D14N, R17S, K26T.

3. The polypeptide for use according to any one of claims 1-2, wherein the amino acid sequence is selected from the group of SEQ ID NOs: 7, 6, 5, and 8, preferably SEQ ID NO: 7.

4. The polypeptide for use according to any one of claims 1-3, wherein the polypeptide is C-terminally modified, such as amidated, and/or N-terminally modified, such as acetylated.

5. The polypeptide for use according to any one of claims 1-4, wherein the polypeptide blocks complex formation between a surface protein of the coronavirus and a receptor of a target cell.

6. The polypeptide for use according to any one of claims 1-5, wherein the polypeptide binds to a receptor of a target cell, preferably wherein the receptor of a target cell is membrane receptor of a target cell, preferably wherein the membrane receptor of a target cell is ACE-2 receptor.

7. The polypeptide for use according to any one of claims 1-6, wherein the polypeptide prevents cell penetration of the coronavirus, preferably wherein the coronavirus is selected from the group of SARS-CoV-2, SARS-CoV-1, HCoV-NL63, and related Coronaviridae, preferably SARS-CoV-2, preferably wherein the SARS-CoV-2 is a SARS-CoV-2 variant selected from the group of ancestral strain, alpha, beta, gamma, delta (kappa), omicron and subvariants thereof, preferably wherein the subvariant of the SARS-CoV-2 variant omicron is selected from the BA.1, BA.2, BA.3, BA.3, BA.4, XBB, JN.1, KP.3, BQ, XE and KP.2.

8. The polypeptide for use according to any one of claims 1-7, wherein the polypeptide is to be administered in a therapeutically effective amount, preferably wherein the therapeutically effective amount is in the range of about 33 to about 800 mg/L, equivalent to about 10 to about 244 µM.

9. The polypeptide for use according to any one of claims 1-8, wherein the polypeptide is to be administered parenterally or orally, preferably wherein the parenteral administration is by a spray, such as a nasal or oral spray, by inhalation, such as vapor, powder, or aerosol inhalation, or by injection, preferably by nasal spray or aerosol inhalation.

10. The polypeptide for use according to any one of claims 1-9, wherein the polypeptide is to be administered in combination with one or more further antiviral agent(s), preferably wherein the one or more further antiviral agent(s) is/are selected from the group of: mRNA vaccines, adenovirus vector vaccines, inactivated virus vaccines, subunit vaccines, monoclonal antibody therapies and other small molecule treatments, preferably, wherein the one or more further antiviral agent(s) is selected from the group of Remdesivir, Nirmatrelvir, Ritonavir, Dexamethasone, and Tocilizumab.

11. The polypeptide for use according to claim 10, wherein the further antiviral agent is an antibody.

12. The polypeptide for use according to claim 11, wherein the antibody binds a coronavirus, preferably wherein the antibody binds the spike (S) protein of the coronavirus, preferably wherein the antibody binds the RBD of the coronavirus spike (S) protein, preferably wherein the coronavirus is selected from the group of SARS-CoV-2, SARS-CoV-1, HCoV-NL63, and related Coronaviridae, preferably SARS-CoV-2 or, wherein the antibody binds the ACE-2 receptor.

13. A polypeptide, as defined in any one of claims 2-12.

14. A polypeptide as defined in any one of claims 1-12 for use as a medicament.

15. A composition comprising a polypeptide as defined in any one of claims 1-12 as an active ingredient, preferably wherein the composition is for use as a medicament, even more preferably wherein the composition is for use in treatment and/or prevention of coronavirus infection.
